# EUROPÄISCHE PATENTSCHRIFT

(11) **EP 4 573 898 B1**
(45) Veröffentlichungstag und Bekanntmachung des Hinweises auf die Patenterteilung: **25.11.2026**
(21) Anmeldenummer: 25166936.2
(22) Anmeldetag: 04.08.2020
(51) Int. Cl.: A01M 7/00

(54) **VORHERSAGE VON PFLANZENSCHUTZMITTEL-RÜCKSTÄNDEN IN ERNTEPRODUKTEN**
PREDICTION OF RESIDUES OF PLANT PROTECTION AGENTS IN HARVESTED PRODUCTS
PRÉDICTION DE RÉSIDUS D'AGENTS PHYTOSANITAIRES DANS DES PRODUITS DE RÉCOLTE

(30) Priorität: 07.08.2019 EP 19190498
(43) Veröffentlichungstag der Anmeldung: 25.06.2025
(62) Dokumentnummer(n) der früheren Anmeldung(en) nach Art. 76 EPÜ: 20747000.6 / 4 009 784
(73) Patentinhaber: Bayer CropScience Schweiz AG, 4052 Basel (CH)
(72) Erfinder: WIRTZ, Kai, 40593 Düsseldorf (DE); BECKER, Rolf Christian, 51399 Burscheid (DE); GOERLITZ, Linus, 51427 Bergisch Gladbach (DE); SCHAEFER, Dirk, 50171 Kerpen (DE); WEYSSER, Fabian, 51371 Leverkusen (DE)
(74) Vertreter: BIP Patents

(56) Entgegenhaltungen:
- WO-A1-2017/042843
- WO-A2-2013/143968
- JP-A- 2005 085 059
- US-B1- 9 563 852

## Beschreibung

Die vorliegende Erfindung befasst sich mit der Vorhersage von Pflanzenschutzmittel-Rückständen in solchen Pflanzen oder Pflanzenteilen, die zum menschlichen und/oder tierischen Verzehr gedacht sind, vorzugsweise in Gemüse und/oder Obst. Gegenstände der vorliegenden Erfindung sind ein Verfahren, eine Vorrichtung, ein System und ein Computerprogrammprodukt zur Vorhersage von Pflanzenschutzmittel-Rückständen.

CN105675807A offenbart eine Bewertung von Atrazin-Rückständen unter Verwendung eines neuronalen BP-Netzwerks (Backpropagation).

M. Calliera et al. offenbaren eine Vorhersage von Daten über Rückstände von Agrochemikalien auf Obst mit Hilfe eines Informatiksystems (PARDIS-Modell) (Pest Manag Sci 64:981-988 (2008)).

N. Pang et al. offenbaren eine wetterabhängige Dynamik der Herbizide Florasulam, Carfentrazoneethyl, Fluroxypyr-meptyl und Fluroxypyr in Weizenfeldern durch Feldstudien und rechnerische Simulation (Chemosphere 165 (2016) 320-328).

Pflanzenschutzmittel-Rückstände sind Rückstände von Wirkstoffen, die dem Pflanzenschutz dienten, also während der Erzeugung von Pflanzen eingesetzt wurden, und im Endprodukt nachweisbar sind.

Der Einsatz von Pflanzenschutzmitteln ist in vielen Ländern gesetzlich geregelt und soll eine Gefährdung der Gesundheit von Tier und Mensch durch Pflanzenschutzmittel ausschließen. Spezifische Anwendungsvorschriften (Wartezeiten bzw. zeitliche Abstände zwischen letzter Anwendung und Ernte, anzuwendende Menge, Anwendungsbeschränkungen) berücksichtigen die Stoffeigenschaften und nehmen so Einfluss auf das Entstehen und die Höhe von Rückständen.

Gemäß §9 (1) des deutschen Lebensmittel-, Bedarfsgegenstände- und Futtermittelgesetzbuches (Lebensmittel- und Futtermittelgesetzbuch - LFGB) werden Lebensmittel vom Handelsverkehr ausgeschlossen, wenn sie Pflanzenschutzmittel enthalten, die bestimmte für die einzelnen Stoffe festgesetzte Höchstmengen überschreiten.

Die Rückstandshöchstgehalte für Pflanzenschutzmittel in konventionellen Lebens- und Futtermitteln sind seit 1. September 2008 durch das Inkrafttreten der Verordnung (EG) Nr. 396/2005 in allen europäischen Mitgliedsstaaten harmonisiert. Weltweit einheitliche Grenzwerte gibt es jedoch nicht.

Einige Handelsketten (Händler) fordern von ihren Produzenten pflanzliche Lebensmittel mit Pflanzenschutzmittel-Rückständen, die (deutlich) unterhalb der gesetzlichen Bestimmungen liegen. Dabei können die Forderungen einzelner Handelsketten voneinander variieren.

Pflanzenschutzmittel-Rückstände können beispielsweise in Laboren bestimmt und quantifiziert werden. Es gibt zahlreiche Dienstleister, die Pflanzenschutzmittel-Rückstände in Lebens- und Futtermitteln im Auftrag bestimmen.

WO2017/042843A1 offenbart ein agronomisches Verfahren zur Erzeugung von Gemüse und Pilzen. Das Verfahren hat zum Ziel, Produkte mit einer minimalen Menge an Pestizid-Rückständen zu erzeugen. Ein Schritt des Verfahrens ist das wiederholte Messen von Pflanzenschutzmittel-Rückständen in zeitlicher Nähe zur Ernte.

Für einen Erzeuger von pflanzlichen Lebensmitteln und Futtermitteln wäre es vorteilhaft, bereits während des Anbaus der Pflanzen Informationen darüber zu erhalten, welche Mengen an Pflanzenschutzmittel-Rückständen in den geernteten Produkten zu erwarten sind, wenn der Erzeuger ein definiertes Pflanzenschutzmittel gemäß einem definierten Applikationsprogramm verwendet. Der Erzeuger wäre dann in der Lage, durch Änderungen des Pflanzenschutzmittels und/oder des Applikationsprogramms die Rückstandsmenge des Pflanzenschutzmittels in dem Ernteprodukt in gewissem Umfang zu steuern. Ferner wäre es für einen solchen Erzeuger vorteilhaft zu wissen, bei welchen Rückstandswerten welche Handelsketten seine Erzeugnisse abnehmen und an Endkunden weiterverkaufen würden.

Diese Aufgabe wird durch den Gegenstand des unabhängigen Patentanspruchs gelöst. gelöst. Bevorzugte Ausführungsformen finden sich in den abhängigen Patentansprüchen, den Zeichnungen und in der vorliegenden Beschreibung.

Der erste Gegenstand der vorliegenden Erfindung ist ein System umfassend
- ein erstes Computersystem umfassend eine Eingabeeinheit, eine erste Steuer- und Berechnungseinheit, eine erste Sende- und Empfangseinheit und eine Ausgabeeinheit,
- ein zweites Computersystem umfassend eine zweite Steuer- und Berechnungseinheit und eine zweite Sende- und Empfangseinheit,
dadurch gekennzeichnet, dass die erste Steuer- und Berechnungseinheit konfiguriert ist, die Eingabeeinheit zu veranlassen, folgende Eingabeinformationen zu empfangen und/oder zu ermitteln:
- Informationen zu einer Kulturpflanze, die angebaut wird,
- Informationen zum Anbau der Kulturpflanze,
- Informationen zu einem Pflanzenschutzmittel, das beim Anbau der Kulturpflanze verwendet worden ist und/oder dessen Verwendung geplant ist, wobei es sich bei dem Pflanzenschutzmittel um ein Herbizid, ein Fungizid oder ein anderes Pestizid handelt,
- Informationen zur Applikation des Pflanzenschutzmittels,

wobei die erste Steuer- und Berechnungseinheit konfiguriert ist, die erste Sende- und Empfangseinheit zu veranlassen, die Eingabeinformationen über ein Netzwerk an das zweite Computersystem zu übermitteln,
wobei die zweite Steuer- und Berechnungseinheit konfiguriert ist, die zweite Sende- und Empfangseinheit zu veranlassen, die Eingabeinformationen über das Netzwerk zu empfangen,
wobei die zweite Steuer- und Berechnungseinheit konfiguriert ist, eine Menge eines Rückstands des Pflanzenschutzmittels in und/oder auf den zum menschlichen und/oder tierischen Verzehr gedachten Teilen der Kulturpflanze vorzugsweise zum Zeitpunkt der Ernte auf Basis der Eingabeinformationen zu berechnen,
wobei das Berechnen der Menge des Rückstands des Pflanzenschutzmittels mindestens anhand der folgenden Eingabeinformationen erfolgt:
   - angebaute Kulturpflanze
   - zu verwendendes oder verwendetes Pflanzenschutzmittel
   - Aufwandmenge des zu verwendenden oder verwendeten Pflanzenschutzmittels
   - Menge der vorliegenden Biomasse und/oder Fruchtmasse der angebauten Kulturpflanze zum Zeitpunkt der Applikation des Pflanzenschutzmittels
   - Länge der Zeitspanne zwischen dem Zeitpunkt der Applikation und dem Zeitpunkt der Ernte,
wobei das Berechnen der Menge des Rückstands des Pflanzenschutzmittels mit einem Regressionsmodell erfolgt, das mit empirischen Daten trainiert worden ist, die Menge des Rückstands des Pflanzenschutzmittels anhand von Informationen vorherzusagen, wobei die Informationen umfassen:
   - angebaute Kulturpflanze
   - Land oder Region, in der die Kulturpflanze angebaut wird oder Menge der vorliegenden Biomasse und/oder Fruchtmasse der angebauten Kulturpflanze zum Zeitpunkt der Applikation des Pflanzenschutzmittels
   - zu verwendendes oder verwendetes Pflanzenschutzmittel
   - Aufwandmenge des zu verwendenden oder verwendeten Pflanzenschutzmittels
   - Länge der Zeitspanne zwischen dem Zeitpunkt der Applikation und dem Zeitpunkt der Ernte,
wobei die zweite Steuer- und Berechnungseinheit konfiguriert ist, die zweite Sende- und Empfangseinheit zu veranlassen, die Menge des Rückstands über das Netzwerk an das erste Computersystem zu übermitteln,
wobei die erste Steuer- und Berechnungseinheit konfiguriert ist, die erste Sende- und Empfangseinheit zu veranlassen, die Menge des Rückstands über das Netzwerk zu empfangen,
wobei die erste Steuer- und Berechnungseinheit konfiguriert ist, die Ausgabeeinheit zu veranlassen die Menge des Rückstands gegenüber einem Nutzer auszugeben.

Die Erfindung wird nachstehend näher erläutert, ohne zwischen den Gegenständen Verfahren, Vorrichtung, System, Computerprogrammprodukt zu unterscheiden. Die nachfolgenden Erläuterungen sollen vielmehr für alle Gegenstände in analoger Weise gelten, unabhängig davon, in welchem Kontext (Verfahren, Vorrichtung, System, Computerprogrammprodukt) sie erfolgen.

Wenn in der vorliegenden Beschreibung oder in den Patentansprüchen Schritte in einer Reihenfolge genannt werden, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden können; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen dar.

Die vorliegende Erfindung wird mit Hilfe eines Computersystems oder mehrerer Computersysteme ausgeführt.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Geräte können zur Ausführung der Erfindung genutzt werden.

Die Eingaben in den Computer erfolgen über Eingabemittel wie beispielsweise eine Tastatur, eine Maus, ein Mikrofon, ein Netzwerkanschluss, ein externer Datenspeicher und/oder dergleichen. Als Eingabe soll auch die Auswahl eines Eintrags aus einem virtuellen Menü oder einer virtuellen Liste oder das Anklicken eines Auswahlkästchens und dergleichen verstanden werden. Ausgaben erfolgen üblicherweise über einen Bildschirm (Monitor), auf einem Drucker, über Lautsprecher und/oder durch Speicherung auf einem Datenspeicher.

Die erfindungsgemäße Vorrichtung kann als ein solches Computersystem ausgeführt sein. Ferner kann das erfindungsgemäße System mehrere solcher Computersysteme umfassen.

Die vorliegende Erfindung dient der Vorhersage der Rückstandsmenge eines Pflanzenschutzmittels in und/oder auf den zum menschlichen und/oder tierischen Verzehr gedachten Teilen einer Kulturpflanze vorzugsweise zum Zeitpunkt der Ernte.

Die Vorhersage wird für eine spezifische Kulturpflanze getroffen. Unter dem Begriff "Kulturpflanze" wird eine Pflanze verstanden, die durch das Eingreifen der Menschen zielgerichtet als Nutzpflanze angebaut wird. Teile der angebauten Kulturpflanze sind zum menschlichen und/oder tierischen Verzehr geeignet. In einer bevorzugten Ausführungsform handelt es sich bei der Kulturpflanze um eine Obstpflanze oder eine Gemüsepflanze.

Die Vorhersage erfolgt auf Basis von Informationen, die die Kulturpflanze, das Pflanzenschutzmittel und Anbaubedingungen (näher) spezifizieren. Diese Informationen werden in dieser Beschreibung auch als "Eingabeinformationen" bezeichnet. Dabei soll der Begriff "Eingabeinformationen" nicht dahingehend verstanden werden, dass diese Informationen sämtlich von einem Nutzer in das erfindungsgemäße Computersystem eingeben werden. Vielmehr kann ein Teil der Eingabeinformationen auch von dem erfindungsgemäßen Computersystem (auf Basis von eingegebenen Informationen) ermittelt werden (wie nachfolgend näher erläutert wird). Insofern bezieht sich der Begriff "Eingabe" eher darauf, dass die Eingabeinformationen als Eingabe in die Berechnung einer Menge eines Rückstands eines Pflanzenschutzmittels einfließen.

In der vorliegenden Beschreibung wird ferner der Begriff "Spezifizieren" verwendet. Je nachdem, auf welche Weise ein Gegenstand "spezifiziert" wird, kann der Begriff "Spezifizieren" die Bedeutungen "Eingeben", "Ermitteln", "Auswählen (=Selektieren)", "Vorselektieren", "Berechnen" und/oder "Ableiten" haben.

In einem ersten Schritt wird die Kulturpflanze spezifiziert. Dies erfolgt vorzugsweise dadurch, dass ein Nutzer den Namen der Kulturpflanze oder den Namen der Sorte der Kulturpflanze oder einen Code für die Kulturpflanze/Kulturpflanzensorte (z.B. nach dem Internationalen Code der Nomenklatur der Kulturpflanzen, kurz ICNCP) in ein Computersystem eingibt oder die entsprechende Information zu der Kulturpflanze aus einer Liste oder einem Menü auswählt oder eine Kulturpflanze anhand einer bildhaften Darstellung der Kulturpflanze (z.B.: einem Foto oder eine Grafik) auswählt.

Denkbar ist auch, dass die Kulturpflanze oder das Gefäß, in dem sich die Kulturpflanze befindet, oder ein Beet, in dem die Kulturpflanze angebaut wird, oder ein Katalog, in dem die Kulturpflanze aufgeführt ist, oder eine Verpackung für die Kulturpflanze oder für Saatgut zum Anbau der Kulturpflanze mit einem maschinenlesbaren Code ausgestattet ist, der eine Information zu der Kulturpflanze bereitstellt. Die Spezifizierung der Kulturpflanze kann in einem solchen Fall darin bestehen, dass der maschinenlesbare Code mit einem geeigneten Lesegerät ausgelesen und die ausgelesene Information zu der Kulturpflanze mittels des Lesegeräts an das erfindungsgemäße Computersystem übertragen wird. Bei einem solchen maschinenlesbaren Code kann es sich beispielsweise um einen optoelektronisch lesbaren Code (z.B. Strichcode, 2D-Cod (z.B. DataMatrix- oder QR-Code) oder um einen elektronisch in einem Halbleiterspeicher (z.B. einem RFID-Chip) gespeicherten Code handeln.

Denkbar ist auch, dass das erfindungsgemäße Computerprogrammprodukt lediglich für eine einzige Kulturpflanze bestimmt ist, das heißt, lediglich die Menge eines Rückstands eines Pflanzenschutzmittels für eine einzige Kulturpflanze oder Kulturpflanzensorte berechnen/vorhersagen kann. In einem solchen Fall besteht der Schritt "Spezifizieren einer Kulturpflanze" darin, dass ein Nutzer das entsprechende Computerprogrammprodukt auswählt, um Rückstandsmengen eines oder mehrerer Pflanzenschutzmittel in der Kulturpflanze, für die das Computerprogramm bestimmt ist, zu berechnen.

Die Kulturpflanze ist vorzugsweise ausgewählt aus der folgenden Liste: Erdbeere, Tomate, Gurke, Paprika, Radies, Rettich, Kohlrabi, Möhre, Sellerie, Fenchel, Patinake, Erbse, Bohne, Spargel, Spinat, Mangold, Artischocke, Schwarzwurzel, Kopfsalat, Eissalat, Schnittsalat, Endivie, Cicorée, Aubergine, Kürbis, Melone, Zucchini, Feldsalat, Zuckerrübe, Rhabarber, Weißkohl, Rotkohl, Grünkohl, Rosenkohl, Blumenkohl, Broccoli, Himbeere, Brombeere, Holunder, Kirsche, Apfel, Birne, Weintraube, Pflaume.

In einem weiteren Schritt werden Informationen zum Anbau der Kulturpflanze (in dieser Beschreibung auch als Anbauparameter oder Anbaubedingungen bezeichnet) spezifiziert.

Die Anbauparameter können angeben, wo und/oder unter welchen Bedingungen die Kulturpflanze angebaut wird. Es kann zum Beispiel spezifiziert werden, ob die Kulturpflanze im Freien, in einem Folientunnel (wie zum Beispiel bei Erdbeeren praktiziert), in einem Gewächshaus oder dergleichen angebaut werden. Durch den weitgehend fehlenden Witterungseinfluss (z.B. Regen) können Rückstandsmengen bei Pflanzen, die im Gewächshaus kultiviert werden, höher als bei einer Kultivierung im Freiland sein.

Es kann spezifiziert werden, ob eine künstliche Bewässerung erfolgt, ggf. können die Bewässerungsmengen spezifiziert werden.

Insbesondere für den Fall, dass es sich um ein Gewächshaus handelt, kann der Nutzer vorzugsweise die Bedingungen, die im Gewächshaus herrschen, spezifizieren, wie beispielsweise die Temperatur (Luft, Boden), Feuchtigkeit (Luft, Boden) sowie Kohlendioxidgehalt in der Luft, zum Beispiel in Form eines zeitlichen Verlaufs über den Tag / die Nacht, Maximalwert, Minimalwert, Mittelwert (z.B. arithmetischer Mittelwert (Durchschnitt)), Varianz, Temperatursummen, Strahlungssummen und/oder dergleichen.

Die oben genannten Bedingungen (Temperaturen, Feuchtigkeit, Kohlendioxidgehalt, Strahlungsmengen) können natürlich auch für Kulturpflanzen spezifiziert werden, die auf dem freien Feld oder in einem Folientunnel angebaut werden. Vorzugsweise wird die Witterung während der Vegetationsperiode der Kulturpflanze erfasst. Dabei können folgende Werte erfasst werden: Lufttemperatur, Luftfeuchtigkeit, Luftdruck, Windgeschwindigkeit, Niederschlagsart, Niederschlagsmenge, Sonneneinstrahlung und dergleichen. Dabei können zum Beispiel Tageshöchst- und Tagesmittelwerte (z.B. arithmetische Mittelwerte) erfasst werden. Die Witterungsverhältnisse können auf der einen Seite einen Einfluss auf das Abbauverhalten eines Wirkstoffs ausüben und auf der anderen Seite das Wachstumsverhalten der Kulturpflanzen bestimmen.

Vorzugsweise wird die geografische Position des Feldes spezifiziert, auf dem die Kulturpflanze angebaut wird. Anhand der geografischen Position des Feldes können dann Werte für die genannten Bedingungen, die während der Anbauperiode herrschen (aktuelle Werte) oder üblicherweise herrschen (Mittelwerte der Vergangenheit) z.B. aus Datenbanken ausgelesen werden.

Unter dem Begriff "Feld" wird ein räumlich abgrenzbarer Bereich der Erdoberfläche verstanden, der landwirtschaftlich genutzt wird, indem auf einem solchen Feld Kulturpflanzen angepflanzt, mit Nährstoffen versorgt und geerntet werden.

Die Spezifizierung der geografischen Position eines Feldes kann beispielsweise darin bestehen, dass dasjenige Land spezifiziert ist, in dem sich das Feld befindet. Es ist auch denkbar, dass eine Region spezifiziert wird, in der sich das Feld befindet. Bei einer solchen Region kann es sich zum Beispiel um eine Region mit einem definierten Klima handeln, das sich von dem Klima benachbarter Regionen unterscheidet. Bei einer Region kann es sich um ein Anbaugebiet für eine spezifische Kulturpflanze handeln (zur Definition eines Anbaugebietes siehe z.B. Journal für Kulturpflanzen, 61 (7). S. 247-253, 2009, ISSN 0027-7479). Bei einer Region kann es sich um einen Boden-Klima-Raum handeln (zur Definition eines Boden-Klima-Raumes siehe z.B. Nachrichtenbl. Deut. Pflanzenschutzd., 59(7), S. 155-161, 2007, ISSN 0027-7479).

Die Spezifizierung der geografischen Position eines Feldes kann ferner darin bestehen, dass die geografischen Koordinaten (Geokoordinaten) mindestens eines Punktes, der in dem Feld liegt oder am Rande des Feldes liegt, angegeben werden. Viele Felder weisen die Form eines Polygons auf. Für ein solches Feld können zur Spezifizierung die Geokoordinaten der Ecken des Polygons angeben werden. Es ist denkbar, dass ein Nutzer zur Spezifizierung der geografischen Position eines Feldes die Feldgrenzen auf einer virtuellen Karte, die auf einem Bildschirm des erfindungsgemäßen Computersystems mit einem Finger oder einem Eingabegerät (z.B. mit einer Maus) einzeichnet.

Es ist denkbar, dass für eine oder mehrere Kulturpflanzen bereits Informationen zum Anbau (z.B. übliche Anbaubedingungen) in einem Datenspeicher hinterlegt sind. Es ist denkbar, dass die erfindungsgemäße Vorrichtung oder das erfindungsgemäße System nach dem Spezifizieren einer Kulturpflanze die für die Kulturpflanze üblichen Anbaubedingungen aus dem Datenspeicher ausliest und die ausgelesenen Werte der weiteren Berechnung der Rückstandsmenge zugrunde legt. Es ist ferner denkbar, dass die Anbaubedingungen durch Spezifizieren der Position festgelegt werden; für definierte Länder und/oder Regionen können in dem Datenspeicher übliche Anbaubedingungen gespeichert sein, die der weiteren Berechnung zugrunde gelegt werden können. Es ist auch denkbar, dass das erfindungsgemäße Computerprogrammprodukt lediglich für eine einzige Kulturpflanze bestimmt ist, die unter vorgegebenen Anbaubedingungen angebaut wird. In einem solchen Fall besteht der Schritt "Spezifizieren der Anbaubedingungen" darin, dass ein Nutzer das entsprechende Computerprogrammprodukt auswählt.

In einem nächsten Schritt wird mindestens ein Pflanzenschutzmittel spezifiziert, das beim Anbau der spezifizierten Kulturpflanze zum Einsatz kommt, d.h. appliziert wird.

Unter dem Begriff "Pflanzenschutzmittel" wird ein Mittel verstanden, das dazu dient, Pflanzen oder Pflanzenerzeugnisse vor Schadorganismen zu schützen oder deren Einwirkung vorzubeugen, unerwünschte Pflanzen oder Pflanzenteile zu vernichten, ein unerwünschtes Wachstum von Pflanzen zu hemmen oder einem solchen Wachstum vorzubeugen, und/oder in einer anderen Weise als Nährstoffe die Lebensvorgänge von Pflanzen zu beeinflussen (z.B. Wachstumsregler). Besagte Wachstumsregler dienen zum Beispiel der Erhöhung der Standfestigkeit bei Getreide durch Verkürzung der Halmlänge (Halmverkürzer oder besser Internodienverkürzer), Verbesserung der Bewurzelung von Stecklingen, Verringerung der Pflanzenhöhe durch Stauchung im Gartenbau oder der Verhinderung der Keimung von Kartoffeln. Wachstumsregler sind üblicherweise Phytohormone oder deren synthetischen Analoge. Beispiele für weitere Pflanzenschutzmittel sind Herbizide, Fungizide und andere Pestizide (z.B. Insektizide, Nematizide, Molluskizide und dergleichen).

Ein Pflanzenschutzmittel enthält üblicherweise einen Wirkstoff oder mehrere Wirkstoffe. Als "Wirkstoffe" werden Substanzen bezeichnet, die in einem Organismus eine spezifische Wirkung haben und eine spezifische Reaktion hervorrufen. Bei einem solchen Wirkstoff kann es sich um einen synthetisch hergestellten (chemischen) Wirkstoff oder einen aus einem Organismus gewonnenen (biologischen) Wirkstoff handeln. Auch Kombinationen sind denkbar. Üblicherweise enthält ein Pflanzenschutzmittel einen Trägerstoff zum Verdünnen des einen oder der mehreren Wirkstoffe. Daneben sind Additive wie Konservierungsmittel, Puffer, Farbstoffe und dergleichen denkbar. Ein Pflanzenschutzmittel kann fest, flüssig oder gasförmig vorliegen.

Unter einem "Schadorganismus" wird ein Organismus verstanden, der beim Anbau von Kulturpflanzen in Erscheinung treten und die Kulturpflanze schädigen, die Ernte der Kulturpflanze negativ beeinflussen oder mit der Kulturpflanze um natürliche Ressourcen konkurrieren kann. Beispiele für derartige Schadorganismen sind Unkräuter, Ungräser, tierische Schädlinge wie beispielsweise Käfer, Raupen und Würmer, Pilze und Krankheitserreger (z.B. Bakterien und Viren). Auch wenn Viren aus biologischer Sicht nicht zu den Organismen zählen, sollen sie in dieser Beschreibung dennoch unter den Begriff Schadorganismus fallen.

Unter dem Begriff "Unkraut" (Mehrzahl: Unkräuter) werden Pflanzen der spontanen Begleitvegetation in Kulturpflanzenbeständen, Grünland oder Gartenanlagen verstanden, die dort nicht gezielt angebaut werden und z.B. aus dem Samenpotential des Bodens oder über Zuflug zur Entwicklung kommen. Der Begriff ist nicht auf Kräuter im eigentlichen Sinne beschränkt, sondern umfasst auch Gräser, Farne, Moose oder holzige Pflanzen. Im Bereich des Pflanzenschutzes wird häufig auch der Begriff "Ungras" (Mehrzahl: Ungräser) benutzt, um eine Abgrenzung zu den krautigen Pflanzen zu verdeutlichen. Im vorliegenden Text wird der Begriff Unkraut als Oberbegriff verwendet, der Ungras mit erfassen soll, es sei denn, es wird auf spezifische Unkräuter oder Ungräser Bezug genommen.

Unter dem Begriff "Bekämpfung" werden eine Verhinderung des Befalls eines Feldes / einer Kulturpflanze oder einem Teil davon mit einem oder mehreren Schadorganismen und/oder eine Verhinderung der Ausbreitung von einem oder mehreren Schadorganismen und/oder eine Reduzierung der Menge an vorhandenen Schadorganismen verstanden.

Die Spezifizierung eines Pflanzenschutzmittels ist aus zwei Gründen wichtig: Zum einen wird/werden durch die Spezifizierung des Pflanzenschutzmittels auch der Stoff/die Stoffe spezifiziert, dessen/deren Rückstandsmenge(n) erfindungsgemäß vorhergesagt werden soll(en). Bei dem Stoff, für den eine Rückstandsmenge vorhergesagt werden soll, handelt es sich üblicherweise um den Wirkstoff des Pflanzenschutzmittels und/oder um ein Abbauprodukt des Wirkstoffs, das (ebenfalls) eine biologische Wirkung in einem Organismus ausübt. Zum anderen wird das Abbauverhalten (und damit auch die Rückstandsmenge) maßgeblich durch die chemische Struktur des Wirkstoffs und ggf. seine Formulierung in dem Pflanzenschutzmittel bestimmt. Für viele Wirkstoffe liegen Modelle zum Abbauverhalten vor (siehe z.B. Environ. Sci. Technol. 2019, 53, 5838-5847; Soulas, G. & Lagacherie, B. Biol Fertil Soils (2001) 33: 551. https://doi.org/10.1007/s003740100363; Beulke, S. & Brown, C.D. Biol Fertil Soils (2001) 33: 558. https://doi.org/10.1007/s003740100364; Pagel, Holge, et al., Biogeochemistry, vol. 117, 2014, pp. 185-204., www.jstor.org/stable/24716853; https://www.epa.gov/pesticide-science-and-assessing-pesticide-risks/guidance-calculaterepresentative-half-life-values). Oftmals liegt ein exponentieller Abbau vor; das heißt, die Menge an ausgebrachtem Wirkstoff nimmt exponentiell mit der Zeit ab. Ferner bestimmen die physikalischchemischen Eigenschaften des Wirkstoffes, in welchem Ausmaß der Wirkstoff über das Leitungsbahnensystem der Pflanzen in essbare Anteile transportiert wird.

Die Spezifizierung eines Pflanzenschutzmittels kann beispielsweise auf Basis des Produktnamens des Pflanzenschutzmittels oder eines anderen Namens oder der chemischen Formel für einen Wirkstoff, der in dem Pflanzenschutzmittel enthalten ist, erfolgen.

Denkbar ist auch, dass ein Pflanzenschutzmittel anhand eines Namens und/oder einer bildhaften Darstellung (z.B. einem Foto des Produkts) aus einer Liste ausgewählt wird.

Denkbar ist auch, dass die Verpackung des Pflanzenschutzmittels einen maschinenlesbaren Code enthält, der Informationen zu dem Pflanzenschutzmittel bereitstellt und der durch ein geeignetes Lesegerät ausgelesen werden kann. Bei dem maschinenlesbaren Code kann es sich, wie bereits oben beschrieben, um einen optoelektronisch lesbaren Code und/oder einen elektronisch in einem Halbleiterspeicher (z.B. eines RFID-Tags) gespeicherten Code handeln.

Es ist denkbar, dass mehrere Pflanzenschutzmittel spezifiziert werden, die gleichzeitig oder zu verschiedenen Zeitpunkten appliziert werden/werden sollen.

Denkbar ist auch, dass das erfindungsgemäße Computerprogrammprodukt lediglich für ein einziges Pflanzenschutzmittel bestimmt ist, das heißt, lediglich die Menge eines Rückstands eines spezifischen Pflanzenschutzmittels berechnen/vorhersagen kann. In einem solchen Fall besteht der Schritt "Spezifizieren eines Pflanzenschutzmittels" darin, dass ein Nutzer das entsprechende Computerprogrammprodukt auswählt, um Rückstandsmengen desjenigen Pflanzenschutzmittels in einer Kulturpflanze zu ermitteln, für das das Computerprogramm bestimmt ist.

Vorzugsweise sind die erfindungsgemäße Vorrichtung und das erfindungsgemäße System so konfiguriert, dass sie auf Basis der Information zur angebauten Kulturpflanze und/oder auf Basis der Information zur geografischen Position des Feldes und/oder auf Basis der Anbaubedingungen, eine Vorauswahl für das mindestens eine Pflanzenschutzmittel treffen. Der Begriff "Vorauswahl" im Zusammenhang mit einem Pflanzenschutzmittel bedeutet, dass von der Vorrichtung / dem System aus einer Liste an Pflanzenschutzmitteln diejenigen Pflanzenschutzmittel selektiert und dem Nutzer angezeigt werden, die üblicherweise bei der spezifizierten Kulturpflanze verwendet werden und/oder üblicherweise zur Bekämpfung von Schadorganismen verwendet werden, die bei der spezifizierten Kulturpflanze auftreten können, und/oder es werden diejenigen Pflanzenschutzmittel selektiert, die gegen Schadorganismen wirken, die unter den Bedingungen, die an der geografischen Position des spezifizierten Feldes oder in dem Gewächshaus herrschen, in Erscheinung treten können. Unter den vorselektierten Pflanzenschutzmitteln kann dann ein Nutzer eines (oder mehrere) auswählen, für die eine Berechnung einer Rückstandsmenge erfolgen soll. Denkbar ist auch, dass bei der Vorselektion nur ein Pflanzenschutzmittel in Frage kommt, welches dann gegenüber einem Nutzer angezeigt werden kann.

Je nach der geografischen Position, die für das Feld spezifiziert worden ist, kann die Liste an vorselektierten Pflanzenschutzmitteln variieren. Vorzugsweise sind die erfindungsgemäße Vorrichtung und das erfindungsgemäße System so konfiguriert, dass sie lediglich diejenigen Pflanzenschutzmittel vorselektieren und anzeigen, für die in dem Land, in dem sich das jeweilige Feld befindet, eine behördliche Zulassung für das Ausbringen besteht. Entsprechende Informationen zu behördlichen Zulassungen können in einer oder mehreren Datenbanken gespeichert sein.

In einem weiteren Schritt wird die Applikation des Pflanzenschutzmittels vorzugsweise anhand von Applikationsparametern (näher) spezifiziert. Zu den Applikationsparametern zählen die Art der Behandlung, die Aufwandmenge und mindestens ein Zeitpunkt oder mindestens eine Zeitspanne, zu dem / in der das Pflanzenschutzmittel in der spezifizierten Menge appliziert wird/werden soll. Je früher in der Vegetationsperiode der Nutzpflanze eine Behandlung erfolgt, desto geringer ist üblicherweise die Menge der Rückstände.

Dabei ist es denkbar, dass während einer Vegetationsperiode mehrfach (zu unterschiedlichen Zeitpunkten oder in unterschiedlichen Zeitspannen) Pflanzenschutzmittel appliziert wird. Bei einer Mehrfachanwendung können Anzahl der Anwendungen und der zeitliche Abstand zwischen den Anwendungen spezifiziert werden. Die Aufwandmengen können bei einer mehrfachen Ausbringung gleich sein oder variieren. Auch die Art der Behandlung und/oder das jeweils verwendete Pflanzenschutzmittel können variieren. Von großer Bedeutung ist die Zeitspanne zwischen der letzten Applikation eines Pflanzenschutzmittels und der Ernte: im Regelfall werden Pflanzenschutzmittel-Rückstände mit der Zeit abgebaut; je länger also der Zeitpunkt der letzten Applikation her ist, desto geringer ist im Regelfall die Rückstandsmenge.

Bei der Spezifizierung der Art Behandlung kann beispielsweise angegeben werden, ob es sich um eine Behandlung des Saatguts oder der Pflanze in einem definierten Entwicklungsstadium handelt. Behandlungen direkt auf das Erntegut führen im Normalfall zu höheren Rückstandsmengen als beispielsweise Saatgutbehandlungen vor der Aussaat. Ferner kann angegeben werden, welche Pflanzenteile behandeln werden (z.B. Blätter, Früchte und/oder Wurzeln) oder ob eine Bodenbehandlung stattfindet.

Die "Aufwandmenge" ist die zur Bekämpfung von Schadorganismen notwendige Menge eines Pflanzenschutzmittels, die üblicherweise pro Fläche (beim Ausbringen auf dem Feld), pro Raumeinheit (z. B. im Gewächshaus) oder pro Saatgutmenge (z.B. bei der Beizung) angegeben wird. Die Menge kann in Form des Gewichts (z.B. kg) oder in Form des Volumens (z.B. L) angegeben sein. Die Menge kann sich auf die Gesamtmenge des Pflanzenschutzmittel-Produkts oder auf den in dem Pflanzenschutzmittel-Produkt befindlichen Wirkstoff beziehen. Je größer die ausgebrachte Wirkstoffmenge auf die Fläche ist, umso höher ist tendenziell auch die Rückstandsmenge im/auf/am Erntegut.

Falls Wasser zur Verdünnung eines Pflanzenschutzmittels eingesetzt wird, kann auch die Menge des verwendeten Wassers spezifiziert werden.

Die Applikationsparameter können von einem Nutzer in die erfindungsgemäße Vorrichtung oder das erfindungsgemäße System eingegeben werden. Ferner können die Applikationsparameter ganz oder teilweise von der erfindungsmäßen Vorrichtung oder dem erfindungsgemäßen System unter Verwendung der bereits eingegebenen Informationen zur Kulturpflanze, zur geografischen Position des Feldes, zu den Anbaubedingungen, und/oder zum Pflanzenschutzmittel ermittelt werden.

Denkbar ist zum Beispiel, dass für ein Pflanzenschutzmittel von dem Hersteller oder Vertreiber des Pflanzenschutzmittels Informationen bereitgestellt wurden, wann und in welchen Mengen das Pflanzenschutzmittel appliziert werden sollte. Diese Informationen können in einem internen oder externen Datenspeicher, auf den die erfindungsgemäße Vorrichtung / das erfindungsgemäße System zugreifen kann, gespeichert sein. Die Vorrichtung / das System kann also konfiguriert sein, auf Basis der vorliegenden Informationen einen Vorschlag zu generieren, wann das Pflanzenschutzmittel in welchen Mengen ausgebracht werden sollte.

Zeitpunkte / Zeitspannen, zu denen / in denen Pflanzenschutzmittel appliziert werden, können sich beispielsweise (auch) an dem Entwicklungsstadium der Kulturpflanze orientieren. Das Entwicklungsstadium einer Pflanze kann beispielsweise in Form des so genannten BBCH-Codes angegeben werden. Die Abkürzung BBCH steht offiziell für die Biologische Bundesanstalt, das Bundessortenamt und die CHemische Industrie. Die erweiterte BBCH-Skala zur einheitlichen Kodierung der phänologischen Entwicklungsstadien mono- und dikotyler Pflanzen ist eine Gemeinschaftsarbeit der Biologischen Bundesanstalt für Land- und Forstwirtschaft (BBA), des Bundessortenamtes (BSA), des Industrieverbandes Agrar (IVA) und des Instituts für Gemüse u. Zierpflanzenbau Großbeeren/Erfurt. Es ist denkbar, dass ein Nutzer den (erweiterten) BBCH-Code für die angebaute Kulturpflanze in die Vorrichtung / das System eingibt. Denkbar ist ferner, dass die Vorrichtung / das System konfiguriert ist, den BBCH-Code selbst zu berechnen. Es ist beispielsweise denkbar, dass der Nutzer den Zeitpunkt der Anpflanzung / der Aussaat der Kulturpflanze in die Vorrichtung / das System eingegeben hat und die Vorrichtung / das System den BBCH-Code unter Nutzung des Zeitpunkts für die Anpflanzung / die Aussaat berechnet.

Es ist denkbar, dass die erfindungsgemäße Vorrichtung / das erfindungsgemäße System konfiguriert ist, das Entwicklungsstadium der Kulturpflanze mit Hilfe eines Pflanzenwachstumsmodells zu berechnen. Bei einem solchen Pflanzenwachstumsmodell kann es sich beispielsweise um ein mathematisches Modell handeln, welches das Wachstum einer Pflanze in Abhängigkeit von intrinsischen (Genetik) und extrinsischen (Umwelt) Faktoren beschreibt. Einen Überblick über die Erstellung von Pflanzenwachstumsmodellen bieten beispielsweise die Bücher i) "Mathematische Modellbildung und Simulation" von Marco Günther und Kai Velten, erschienen im Wiley-VCH Verlag im Oktober 2014 (ISBN: 978-3-527-41217-4), sowie ii) "Working with Dynamic Crop Models" von Daniel Wallach, David Makowski, James W. Jones und Francois Brun., erschienen 2014 in Academic Press (Elsevier), USA.

Pflanzenwachstumsmodelle existieren für eine Vielzahl an Kulturpflanzen. Das Pflanzenwachstumsmodell simuliert üblicherweise das Wachstum eines Bestands an Kulturpflanzen über einen definierten Zeitraum. Denkbar ist auch, ein Modell auf Basis einer einzelnen Pflanze zu verwenden, das die Energie- und Stoffflüsse in den einzelnen Organen der Pflanze simuliert. Es sind zudem Mischmodelle verwendbar.

Das Wachstum einer Kulturpflanze wird neben den genetischen Merkmalen der Pflanze vornehmlich durch die über die Lebensdauer der Pflanze herrschenden lokalen Witterungen (Quantität und spektrale Verteilung der einfallenden Sonnenstrahlen, Temperaturverläufe, Niederschlagsmengen, Windeintrag), dem Zustand des Bodens sowie das Nährstoffangebot bestimmt.

Auch die bereits (in der Vergangenheit) erfolgten Kulturmaßnahmen und ein etwaig aufgetretener Befall mit Schadorganismen können einen Einfluss auf das Pflanzenwachstum ausüben und können in dem Wachstumsmodell berücksichtigt werden.

Die Pflanzenwachstumsmodelle sind i.d.R. sogenannte dynamische prozessbasierte Modelle (s. "Working with Dynamic Crop Models" von Daniel Wallach, David Makowski, James W. Jones und Francois Brun., erschienen 2014 in Academic Press (Elsevier), USA), können aber auch gänzlich oder teilweise regelbasiert oder statistisch bzw. datengestützt/empirisch sein. Die Modelle sind i.d.R. sogenannte Punktmodelle. Hierbei werden die Modelle i.d.R. so kalibriert, dass der Output die räumliche Repräsentanz des Inputs wiederspiegelt. Ist der Input an einem Punkt im Raum erhoben oder wird für einen Punkt im Raum interpoliert oder geschätzt, so wird i.d.R. angenommen, dass der Modelloutput für das gesamte angrenzende Feld gültig ist. Eine Anwendung von auf Feldebene kalibrierten sog. Punktmodellen auf weitere, i.d.R. gröbere Skalen ist bekannt (siehe z.B.: H. Hoffmann et al.: Impact of spatial soil and climate input data aggregation on regional yield simulations. PLoS ONE 11(4): e0151782. doi:10.1371/journal.pone.0151782). Eine Anwendung dieser sog. Punktmodelle auf mehrere Punkte innerhalb eines Feldes ermöglicht hierbei eine teilflächenspezifische Modellierung. Hierbei werden allerdings räumliche Abhängigkeiten vernachlässigt, z.B. im Bodenwasserhaushalt. Andererseits existieren auch Systeme zur zeitlich-räumlich expliziten Modellierung. Dabei werden räumliche Abhängigkeiten berücksichtigt.

Beispiele für dynamische, prozessbasierte Pflanzenwachstumsmodelle sind Apsim, Lintul, Epic, Hermes, Monica, STICS u.a. Ein Vergleich der Modelle und entsprechende Literatur zu den Modellen kann z.B. der folgenden Veröffentlichung und den darin aufgeführten Verweisen entnommen werden: H. Hoffmann et al.: Impact of spatial soil and climate input data aggregation on regional yield simulations. PLoS ONE 11(4): e0151782. doi: 10.1371/journal.pone.0151782.

In die Modellierung des Pflanzenwachstums können folgende Parameter einfließen:
(a) Wetter: tägliche Niederschlagssummen, Strahlungssummen, Tagesminimum und -maximum der Lufttemperatur sowie Temperatur in Bodennähe sowie Bodentemperatur, Windgeschwindigkeit, u.a.
(b) Boden: Bodentyp, Bodentextur, Bodenart, Feldkapazität, Permanenter Welkepunkt, Organischer Kohlenstoff, mineralischer Stickstoffgehalt, Lagerungsdichte, Van-Genuchten-Parameter, u.a.
(c) Kulturpflanze: Art, Sorte, sortenspezifische Parameter wie z.B. Spezifischer Blattflächenindex, Temperatursummen, maximale Wurzeltiefe, u.a.
(d) Kulturmaßnahmen: Saatgut, Aussaattermin, Aussaatdichte, Aussaattiefe, Düngemittel, Düngemenge, Anzahl an Düngeterminen, Düngetermin, Bodenbearbeitung, Ernterückstände, Fruchtfolge, Distanz zum Feld gleicher Kultur im Vorjahr, Bewässerung, u.a.

Mit Hilfe eines Pflanzenwachstumsmodells kann auch die zu einem Zeitpunkt vorhandene Menge an Biomasse und/oder die Größe der Blattfläche und/oder die Menge an Früchten (Fruchtmasse) und/oder die Zahl vorhandener Triebe und/oder dergleichen berechnet werden. Es ist denkbar, dass eine Pflanze mit mehr Biomasse und/oder einer größeren Blattfläche eine größere Menge an Pflanzenschutzmittel benötigt als eine Pflanze mit weniger Biomasse und/oder einer geringeren Blattfläche. Es ist denkbar, dass die Menge an vorhandener Biomasse - insbesondere in Form von Früchten - zum Zeitpunkt der Applikation eines Pflanzenschutzmittels einen Einfluss auf die Rückstandsmenge ausübt. In einer bevorzugten Ausführungsform der vorliegenden Erfindung fließt daher auch die zu einem Zeitpunkt (insbesondere zum Zeitpunkt der Applikation eines Pflanzenschutzmittels) vorhandene Menge an Biomasse der angebauten Kulturpflanze in die Berechnung einer Rückstandsmenge ein.

Zur Ermittlung und/oder Vorhersage der Menge an vorliegender Biomasse und/oder zur Optimierung von Pflanzenwachstumsmodellen können auch Fernerkundungsdaten hinzugezogen werden. "Fernerkundungsdaten" sind digitale Informationen, die aus der Ferne beispielsweise durch Satelliten von der Erdoberfläche gewonnen wurden. Auch der Einsatz von Luftfahrzeugen (unbemannt (Drohnen) oder bemannt) zur Aufnahme von Fernerkundungsdaten ist denkbar. Durch die Fernerkundungssensoren werden digitale Abbildungen von Bereichen der Erdoberfläche erzeugt, aus denen Informationen über die dort herrschende Vegetation und/oder die dort herrschenden Umweltbedingungen gewonnen werden können (siehe z.B. M. S. Moran et al.: Opportunities and Limitations for Image-Based Remote Sensing in Precision Crop Management, Remote Sensing of Environment (1997) 61: 319-346). Die Daten dieser Sensoren werden über die vom Anbieter zur Verfügung gestellten Schnittstellen bezogen und können optische und elektromagnetische (z.B. Synthetic Aperture Radar SAR) Datensätze verschiedener Verarbeitungsstufen umfassen.

Es können ferner Sensoren im Feld eingesetzt werden, um das Entwicklungsstadium und/oder die vorhandene Biomasse der Kulturpflanzen zu bestimmen. Die Sensoren können stationär im Feld positioniert sein; denkbar ist auch die Ausstattung von Landmaschinen und/oder Robotern, die sich durch das Feld bewegen, mit entsprechenden Sensoren.

Zeitpunkte / Zeitspannen, zu denen / in denen Pflanzenschutzmittel appliziert werden, können sich an den Entwicklungsstadien und/oder der Ausbreitung von Schadorganismen orientieren. Auch für die Entwicklung und/oder Ausbreitung von Schadorganismen sind Vorhersagemodelle verfügbar (siehe z.B.: WO2017/222722A1, WO2018/058821A1, US20020016676, US20180018414A1, WO2018/099220A1). Mit Hilfe solcher Modelle können Zeitpunkte vorhergesagt werden, zu denen ein hohes Risiko eines Befalls der Kulturpflanzen mit Schadorganismen herrschen. Eine Bekämpfung der Schadorganismen mit einem Pflanzenschutzmittel wird vorzugsweise dann vorgenommen, wenn das (errechnete) Risiko eines Befalls besonders hoch ist (zum Beispiel einen definierten Schwellenwert überschreitet). Auch die Modelle zur Vorhersage eines Befalls mit einem Schadorganismus verwenden üblicherweise Wetterdaten und historische Daten.

Die erfindungsgemäße Vorrichtung und das erfindungsgemäße System sind konfiguriert, auf Basis der vorliegenden Informationen (Eingabeinformationen, von den Eingabeinformationen abgeleitete Informationen und/oder anhand der Eingabeinformationen aus einem oder mehrere Datenspeicher(n) ausgelesenen Informationen) die Menge mindestens eines Rückstands eines Pflanzenschutzmittels in und/oder auf der Kulturpflanze oder in und/oder auf einem Teil der Kulturpflanze vorzugsweise zum Zeitpunkt der Ernte zu berechnen. Falls es nach der Ernte (z.B. während einer Lagerung, durch eine Waschung und/oder dergleichen) noch zu einer signifikanten Reduzierung der Rückstandsmenge kommt, kann die Rückstandsmenge (auch) für einen anderen Zeitpunkt als den Zeitpunkt der Ernte berechnet werden und/oder für einen Zustand nach einer spezifischen Behandlung berechnet werden (beispielsweise Waschung, Aussetzen von elektromagnetischer Strahlung, Wärme- oder Kältebehandlung und/oder dergleichen).

Die Berechnung der Menge des mindestens einen Rückstands kann beispielsweise auf Basis von empirisch ermittelten Daten zur Verteilung von Pflanzenschutzmittel in Teilen der Kulturpflanze und zum Abbau von Pflanzenschutzmitteln erfolgen.

In einer besonders bevorzugten Ausführungsform erfolgt die Berechnung der Menge des mindestens eines Rückstands eines Pflanzenschutzmittels mit Hilfe eines Regressionsmodells auf Basis von empirischen Daten. Das Regressionsmodell beschreibt den Zusammenhang zwischen den Eingangsinformationen (z.B. geografische Position des Feldes, Kulturpflanze, Pflanzenschutzmittel und Applikationsparameter) und mindestens einem Ausgangswert (Menge des Rückstands des Pflanzenschutzmittels vorzugsweise zum Zeitpunkt der Ernte).

Das Regressionsmodell kann mittels maschinellen Lernens erstellt werden. Vorzugsweise wird ein solches Modell mittels überwachten Lernens erstellt. Dabei wird das Regressionsmodell trainiert, auf Basis von Eingangsdaten eine Vorhersage für mindestens ein Ausgangsdatum zu treffen. Zum Trainieren und Validieren des Modells können empirische Daten herangezogen werden. Das heißt, es wird für eine Vielzahl an Kulturpflanzen, Anbaubedingungen, Pflanzenschutzmitteln und Applikationsbedingungen empirisch ermittelt, welche Pflanzenschutzmittel-Rückstandsmengen sich beispielsweise zum Erntezeitpunkt ergeben. Aus diesen empirisch gewonnenen Daten lässt sich dann ein entsprechendes Regressionsmodell erstellen.

Bei der Untersuchung von solchen empirischen Daten wurde gefunden, dass sich für eine Vielzahl an Kulturpflanzen und Pflanzenschutzmitteln die Pflanzenschutzmittel-Rückstandsmengen anhand von wenigen Eingangsinformationen vorhersagen lassen. Dabei sind mindestens die folgenden Informationen (als Eingabeinformationen z.B. für das Regressionsmodell) erforderlich: Kulturpflanze, Anbaubedingungen oder eine mit den Anbaubedingungen korrelierende Größe, Pflanzenschutzmittel, Aufwandmenge des Pflanzenschutzmittels und Zeitspanne zwischen der Applikation des Pflanzenschutzmittels und dem Erntezeitpunkt. Eine mit den Anbaubedingungen korrelierende Größe ist beispielsweise die Menge der zum Applikationszeitpunkt vorliegenden Biomasse, die, wie oben beschrieben, mittels Sensoren empirisch ermittelt und/oder durch Berechnung mit Hilfe eines Pflanzenwachstumsmodells berechnet und/oder durch den Nutzer (z.B. nach Augenscheinnahme) spezifiziert werden kann. In einer besonders bevorzugten Ausführungsform erfolgt die Vorhersage der Rückstandsmenge auf Basis der folgenden Informationen (die auch für das Trainieren des entsprechenden Regressionsmodells verwendet werden): angebaute Kulturpflanze, Land oder Region, in der die Kulturpflanze angebaut wird oder Menge der vorliegenden Biomasse und/oder Fruchtmasse der angebauten Kulturpflanze zum Zeitpunkt der Applikation des Pflanzenschutzmittels (oder eine damit korrelierende Größe wie beispielsweise der Durchmesser einer Pflanze oder einer Frucht), zu verwendendes oder verwendetes Pflanzenschutzmittel, Aufwandmenge des zu verwendenden oder verwendeten Pflanzenschutzmittels und Länge der Zeitspanne zwischen dem Zeitpunkt der Applikation und dem Zeitpunkt der Ernte. Besonders vorteilhaft ist es, wenn neben den genannten Eingabeinformationen noch ein oder mehrere der folgenden weiteren Eingabeinformationen zur Witterung während der Anbauphase in die Berechnung einfließen: Sonneneinstrahlung (z.B. in Form von Sonnenstunden), Feuchtigkeit und/oder Temperatur.

Details zur Erstellung eines Regressionsmodells sind im Stand der Technik beschrieben (siehe z.B. Norman Matloff: Statistical Regression and Classification - From Linear Models to Machine Learning, Texts in Statistical Science, CRC Press 2017, ISBN 978-1-4987-1091-6; Pratap Dangeti, Statistics for Machine Learning, Packt Publishing 2017, ISBN 978-1-78829-575-8).

Vorzugsweise handelt es sich um ein nicht-lineares Regressionsmodell (siehe z.B.: Christian Ritz, Jens Carl Streibig: Nonlinear Regression with R, Springer Science & Business Media, 2008, ISBN 9780387096162; R. Russell Rhinehart: Nonlinear Regression Modeling for Engineering Applications: Modeling, Model Validation, and Enabling Design of Experiments, Wiley-ASME Press Series, John Wiley & Sons, 2016, ISBN 9781118597965; Hossein Riazoshams et al.: Robust Nonlinear Regression: with Applications using R, John Wiley & Sons, 2018, ISBN 9781118738061).

In einer bevorzugten Ausführungsform erfolgt die Berechnung der Rückstandsmenge mit Hilfe eines nicht-linearen Regressionsmodells auf Basis der folgenden Eingabeinformationen:
- angebaute Kulturpflanzen
- verwendetes Pflanzenschutzmittel
- Zahl der Applikationen mit dem Pflanzenschutzmittel und applizierte Mengen
- Zeitspanne zwischen der letzten Applikation und dem Erntezeitpunkt
- durchschnittliche Luft- und/oder Bodentemperatur an den jeweiligen Tagen der Applikationen
- durchschnittliche Luftfeuchte an den jeweiligen Tagen der Applikationen
- akkumulierte Strahlungsenergie (Sonnenstrahlung oder künstliche Beleuchtung) an den jeweiligen Tagen der Applikationen
- durchschnittlicher Durchmesser und/oder durchschnittliche Höhe der angebauten Kulturpflanzen an den jeweiligen Applikationstagen.

Die ermittelte Menge des Rückstands kann gegenüber einem Nutzer ausgegeben werden. Die Ausgabe erfolgt beispielsweise in Form von Text und/oder Ziffern und/oder Grafiken auf einem Monitor (Bildschirm) und/oder Drucker der erfindungsgemäßen Vorrichtung / des erfindungsgemäßen Systems.

In einer bevorzugten Ausführungsform wird die ermittelte Rückstandsmenge mit einer Maximalmenge oder mit mehreren Maximalmengen verglichen. Die mindestens eine Maximalmenge ist zum Beispiel eine für das jeweilige Pflanzenschutzmittel behördlich zugelassene Höchstmenge eines Rückstands in einer Kulturpflanze.

Die mindestens eine Maximalmenge kann auch eine von einem Händler für das jeweilige Pflanzenschutzmittel geforderte Höchstmenge eines Rückstands in einer Kulturpflanze sein. Ein "Händler" im Sinne der vorliegenden Erfindung ist eine natürliche oder juristische Person, die Kulturpflanzen oder Teile von Kulturpflanzen von einem Erzeuger ankauft und sie (z.B. an Endkunden (Verbraucher)) weiterverkauft.

Die mindestens eine Maximalmenge kann auch eine von einem Nutzer definierte Menge sein.

Die mindestens eine Maximalmenge kann in einem oder in mehreren Datenspeicher(n) gespeichert sein, auf die die erfindungsgemäße Vorrichtung und das erfindungsgemäße System zugreifen können.

In einer bevorzugten Ausführungsform wird dem Nutzer gegenüber angezeigt, inwieweit die ermittelte Rückstandsmenge eine oder mehrere Maximalmengen über- oder unterschreitet. Es ist denkbar, dass der Nutzer ein Land oder eine Region spezifiziert und dem Nutzer angezeigt wird, ob und/oder inwieweit die ermittelte Rückstandmenge die für das spezifizierte Land oder die spezifizierte Region zugelassene Höchstmenge über- oder unterschreitet. Es ist denkbar, dass der Nutzer mehrere Länder/Regionen spezifizieren kann. Es ist denkbar, dass der Nutzer einen Händler spezifiziert und dem Nutzer angezeigt wird, ob und/oder inwieweit die ermittelte Rückstandmenge die von dem spezifizierten Händler zugelassene Höchstmenge über- oder unterschreitet. Es ist denkbar, dass der Nutzer mehrere Händler spezifizieren kann.

In einer bevorzugten Ausführungsform erfolgt die Ausgabe der ermittelten Menge des Rückstands des Pflanzenschutzmittels in Form einer Prozentzahl und/oder in Form einer grafischen Darstellung der Prozentzahl, wobei die Prozentzahl den prozentualen Anteil der berechneten Rückstandmenge an einer maximalen, vorzugsweise gesetzlich oder behördlich erlaubten Rückstandmenge und/oder einer maximalen, von einem Händler vorgeschriebenen Rückstandmenge angibt. In einer bevorzugten Ausführungsform kann der Nutzer ein Land / eine Region oder mehrere Länder/Regionen auswählen und es wird ihm angezeigt, wie groß die errechnete Rückstandsmenge im Verhältnis zur maximalen, in dem Land / in der Region vorgeschriebenen Rückstandsmenge ist. In einer bevorzugten Ausführungsform kann der Nutzer einen oder mehrere Händler auswählen und es wird ihm angezeigt, wie groß die errechnete Rückstandsmenge im Verhältnis zur maximalen, von dem Händler vorgeschriebenen Rückstandsmenge ist.

In einer bevorzugten Ausführungsform wird die berechnete Rückstandsmenge auf die von einer Behörde vorgeschriebene maximale Rückstandsgrenze ("*maximum residue limit*", "*maximum residue level*", MRL) bezogen. Die maximale Rückstandsgrenze ist die maximal zulässige Rückstandskonzentration. In der EU ist beispielsweise die Europäische Arzneimittel-Agentur (EMA) dafür verantwortlich, Rückstandshöchstgehalte zu empfehlen, die nach ihrer Annahme durch die Europäische Kommission zu rechtsverbindlichen Standards für die Lebensmittelsicherheit werden.

Der Rückstandshöchstgehalt wird normalerweise durch wiederholte Feldversuche (in der Größenordnung von 10) ermittelt, bei denen die Ernte gemäß der guten landwirtschaftlichen Praxis (GAP) behandelt wurde und ein angemessenes Vorernteintervall oder eine angemessene Verweildauer verstrichen ist. Für viele Pestizide liegt der Rückstandshöchstgehalt an ihrer Nachweisgrenze ("*limit of detection*", LOD). Es wird häufig die Quantifizierungsgrenze ("*limit of quantification*", LOQ) anstelle der LOD verwendet. Als Faustregel gilt, dass der LOQ-Wert ungefähr das Zweifache des LOD-Werts beträgt. Für Stoffe, die in keinem der Anhänge der EU-Vorschriften enthalten sind, gilt normalerweise ein Standard-Rückstandshöchstgehalt von 0,01 mg / kg (siehe e.g. https://ec.europa.eu/food/plant/pesticides/max_residue_levels_en). Anstelle oder neben dem MRL-Wert kann die berechnete Rückstandsmenge auch in Bezug zu weiteren üblichen Größen gesetzt werden, wie beispielsweise ARfD, ADI und/oder TDI. Die akute Referenzdosis ("*acute reference dose*", ARfD) ist eine Schätzung der Menge eines Stoffes in Lebensmitteln oder Trinkwasser, die über einen kurzen Zeitraum, normalerweise während einer Mahlzeit oder eines Tages, ohne nennenswertes Gesundheitsrisiko für den Verbraucher aufgenommen werden kann. Die erlaubte Tagesdosis ("acceptable daily intake", ADI) bezeichnet die Dosis einer Substanz, die bei lebenslanger täglicher Einnahme als medizinisch unbedenklich betrachtet wird. Handelt es sich um ungewollte Verunreinigungen, spricht man von einer tolerierten Tagesdosis ("tolerable daily intake", TDI).

In einer bevorzugten Ausführungsform ist die erfindungsgemäße Vorrichtung / das erfindungsgemäße System so konfiguriert, dass zu definierten Zeitpunkten oder bei Eintreten definierter Ereignisse eine Aktualisierung der Berechnung der Rückstandsmenge erfolgt, die einer Änderung der Anbaubedingungen, des Pflanzenschutzmittels und/oder der Applikationsparameter Rechnung trägt. Zum Beispiel kann eine erste Berechnung der Rückstandsmenge auf einem für das jeweilige Land oder für die jeweilige Region, in dem / in der die Kulturpflanze angebaut wird, typischem Klima basieren. Im Verlauf der Vegetationsperiode wird dann die Berechnung an die tatsächlich herrschenden Wetterbedingungen angepasst. In analoger Weise kann eine Aktualisierung der Berechnung auf Basis der tatsächlich durchgeführten Applikationen eines Pflanzenschutzmittels erfolgen. Ferner kann eine Aktualisierung auf Basis von Sensordaten (beispielsweise Fernerkundungsdaten und/oder Felddaten) erfolgen, wobei die Sensordaten beispielsweise Auskunft über den Entwicklungsstand der Kulturpflanze und/oder einen zu erwartenden Ernteertrag geben können.

In einer bevorzugten Ausführungsform ist die erfindungsgenmäße Vorrichtung / das erfindungsgemäße System so konfiguriert, dass der Nutzer das spezifizierte Pflanzenschutzmittel ändern kann und/oder die Applikationsparamater ändern kann, worauf die Menge eines Rückstands des Pflanzenschutzmittels neu berechnet und angezeigt wird. Dies ermöglicht dem Nutzer eine Planung. Er kann evaluieren, wie sich eine Änderung des Pflanzenschutzmittels und/oder der Applikationsparameter auf die Menge des Rückstands auswirkt.

In einer bevorzugten Ausführungsform ist die erfindungsgenmäße Vorrichtung / das erfindungsgemäße System konfiguriert, in Reaktion auf eine Eingabe des Nutzers zur Optimierung der Rückstandsmenge, das Pflanzenschutzmittel und/oder die Applikationsparameter so zu verändern, dass sich eine minimale Rückstandmenge ergibt. Bei einer solchen Optimierung kann der Nutzer ggf. entscheiden, welche Parameter veränderbar und welche unveränderbar sind. Die Vorrichtung / das System ändert dann die veränderbaren Parameter, bis ein Minimum bei der Rückstandsmenge erreicht ist und gibt die geänderten Parameter sowie die berechnete (minimale) Rückstandsmenge gegenüber dem Nutzer aus. Verfahren zur mathematischen Optimierung können den zahlreichen Lehrbüchern zu diesem Thema entnommen werden (siehe z.B. Peter Gritzmann: Grundlagen der Mathematischen Optimierung, Springer Spektrum 2013, ISBN: 978-3-528-07290-2).

Weitere bevorzugte Ausführungsformen der Erfindung sind:
Die Erfindung wird nachstehend anhand von Figuren näher erläutert, ohne die Erfindung auf die in den Figuren gezeigten Merkmale und Merkmalskombinationen beschränken zu wollen.

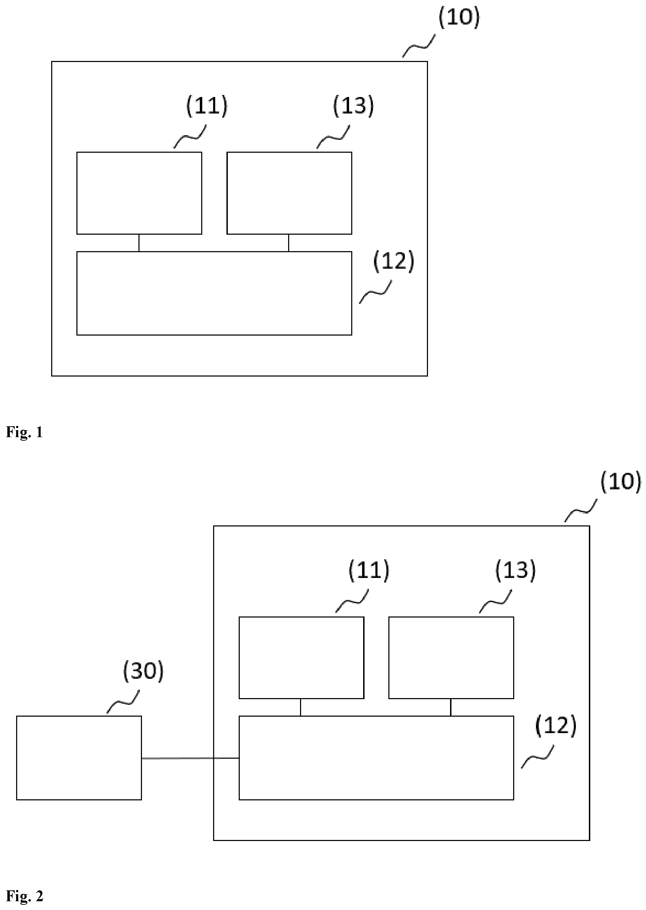

Fig. 1 zeigt beispielhaft und schematisch eine Ausführungsform der erfindungsgemäßen Vorrichtung. Die Vorrichtung (10) umfasst eine Eingabeeinheit (11), eine Steuer- und Berechnungseinheit (12) und eine Ausgabeeinheit (13). Über die Eingabeeinheit (11) kann ein Nutzer Informationen und Steuerbefehle in die Vorrichtung eingeben. Über die Ausgabeeinheit (13) können Informationen gegenüber einem Nutzer ausgegeben, vorzugsweise auf einem Monitor angezeigt werden. Die Steuer- und Berechnungseinheit (12) dient vornehmlich zur Steuerung der Komponenten der Vorrichtung (10), zur Prozessierung der eingegebenen und auszugebenden Informationen und zur Durchführung von Berechnungen und logischen Operationen.
Die Steuer- und Berechnungseinheit (12) ist konfiguriert,
   - die Eingabeeinheit (11) zu veranlassen, folgende Informationen zu empfangen:
      ∘ angebaute Kulturpflanze
      ∘ Anbauparameter zum Anbau der Kulturpflanze
      ∘ zu verwendendes / verwendetes Pflanzenschutzmittel
      ∘ Applikationsparameter zur Applikation des Pflanzenschutzmittels
   - anhand der empfangenen Informationen eine Menge eines Rückstands des Pflanzenschutzmittels in und/oder auf den zum menschlichen und/oder tierischen Verzehr gedachten Teilen der Kulturpflanze vorzugsweise zum Zeitpunkt der Ernte zu berechnen, und
   - die Ausgabeeinheit (13) zu veranlassen die Menge des Rückstands gegenüber einem Nutzer auszugeben.
Fig. 2 zeigt beispielhaft und schematisch eine weitere Ausführungsform der erfindungsgemäßen Vorrichtung. Neben der Eingabeeinheit (11), der Steuer- und Berechnungseinheit (12) und der Ausgabeeinheit (13) wie in Bezug auf Fig. 1 beschrieben, ist die Vorrichtung (10) z.B. über ein Netzwerk mit einem Datenspeicher (30) verbunden. In dem Datenspeicher (30) können beispielsweise Informationen zu Kulturpflanzen (z.B. bevorzugte Anbaubedingungen), zu Pflanzenschutzmitteln (z.B. bevorzugte Applikationsparameter), zum Klima eines Landes oder einer Region, zum Wetter eines Landes oder einer Region, zur Ausbreitung von Schadorganismen in einem Land oder einer Region und dergleichen gespeichert sein. Die Steuer- und Berechnungseinheit (12) kann konfiguriert sein, auf die in dem Datenspeicher (30) gespeicherten Informationen zuzugreifen und diese zur Berechnung der Menge des Rückstands eines Pflanzenschutzmittels zu verwenden. Ferner kann die Steuer- und Berechnungseinheit (12) konfiguriert sein, Informationen in dem Datenspeicher (30) zu speichern. Es ist denkbar, dass es sich bei dem Datenspeicher (30) um mehrere Datenspeicher handelt.

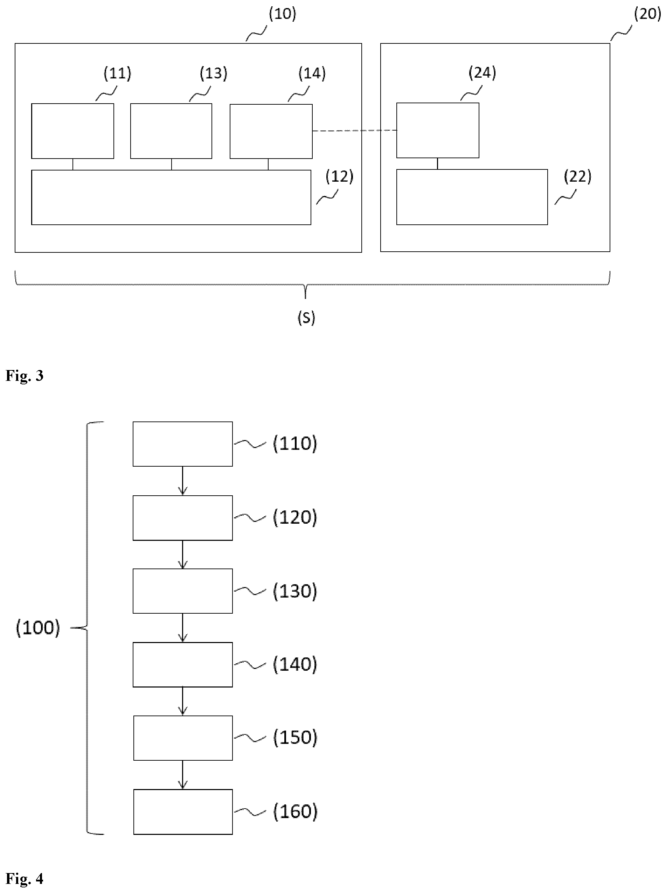

Fig. 3 zeigt beispielhaft und schematisch eine Ausführungsform des erfindungsgemäßen Systems. Das System (S) umfasst ein erstes Computersystem (10) und ein zweites Computersystem (20). Das erste Computersystem (10) ist vorzugsweise als ein Desktop-, Laptop- oder Tablet-Computer oder als ein Smartphone ausgeführt. Das zweite Computersystem (20) ist vorzugsweise als ein Server ausgeführt. Das erste Computersystem (10) wird von einem Nutzer bedient. Das erste Computersystem (10) dient als Kommunikationsschnittstelle zwischen dem Nutzer und dem System (S). Das zweite Computersystem (20) dient dazu, einige Funktionalitäten, die bei der erfindungsgemäßen Vorrichtung von der Steuer- und Berechnungseinheit der Vorrichtung ausgeführt werden, zu übernehmen. Gründe für die Verlagerung von Funktionalitäten auf ein zweites Computersystem können sein:
   - Berechnungen benötigen eine hohe Rechenleistung; diese Berechnungen werden auf einen Server übertragen, der mit der entsprechenden Rechenleistung ausgestattet ist;
   - Berechnungen sollen stets auf den neuesten Versionen von Modellen und aktuellen Daten basieren; diese neuesten Versionen von Modellen und aktuellen Daten werden über einen Server bereitgestellt.

Üblicherweise sind eine Vielzahl von ersten Computersystemen vorhanden, die von unterschiedlichen Nutzern bedient werden, und nur ein zweites Computersystem oder eine geringere (im Vergleich zur Zahl an ersten Computersystemen) Zahl an zweiten Computersystemen, die über ein Netzwerk oder mehrere Netzwerke Ressourcen (Rechenleistung, Daten, Modelle) für die Vielzahl an ersten Computersystemen bereitstellen.

Das erste Computersystem (10) umfasst eine Eingabeeinheit (11), eine erste Steuer- und Berechnungseinheit (12), eine Ausgabeeinheit (13) und eine erste Sende- und Empfangseinheit (14). Das zweite Computersystem (20) umfasst eine zweite Steuer- und Berechnungseinheit (22) und eine zweite Sende- und Empfangseinheit (24). Das erste Computersystem (10) und das zweite Computersystem (20) können über ein Netzwerk Informationen austauschen (dargestellt durch die gestrichelte Linie) zwischen der ersten Sende- und Empfangseinheit (14) des ersten Computersystems (10) und der zweiten Sende- und Empfangseinheit (24) des zweiten Computersystems (20). Das Netzwerk kann ein mobiles Netzwerk umfassen, z.B. eines, das auf dem GSM-, GPRS-, 2G-, 3G-, LTE-, 4G-, 5G-Standard oder einem anderen Standard basiert.

Die erste Steuer- und Berechnungseinheit (12) ist konfiguriert, über die die Eingabeeinheit (11) folgende Eingabeinformationen von einem Nutzer zu empfangen:
- angebaute Kulturpflanze
- Anbauparameter zum Anbau der Kulturpflanze
- zu verwendendes / verwendetes Pflanzenschutzmittel
- Applikationsparameter zur Applikation des Pflanzenschutzmittels.

Die erste Steuer- und Berechnungseinheit (12) ist konfiguriert, die erste Sende- und Empfangseinheit (14) zu veranlassen, die Eingabeinformationen über das Netzwerk an das zweite Computersystem (20) zu übermitteln.

Die zweite Steuer- und Berechnungseinheit (22) ist konfiguriert, die zweite Sende- und Empfangseinheit (24) zu veranlassen, die Eingabeinformationen über das Netzwerk zu empfangen.

Die zweite Steuer- und Berechnungseinheit (22) ist konfiguriert, eine Menge eines Rückstands des Pflanzenschutzmittels in und/oder auf den zum menschlichen und/oder tierischen Verzehr gedachten Teilen der Kulturpflanze vorzugsweise zum Zeitpunkt der Ernte auf Basis der Eingabeinformationen zu berechnen.

Die zweite Steuer- und Berechnungseinheit (22) ist konfiguriert, die zweite Sende- und Empfangseinheit (24) zu veranlassen, die Menge des Rückstands über das Netzwerk an das erste Computersystem (10) zu übermitteln.

Die erste Steuer- und Berechnungseinheit (12) ist konfiguriert, die erste Sende- und Empfangseinheit (14) zu veranlassen, die Menge des Rückstands über das Netzwerk zu empfangen.

Die erste Steuer- und Berechnungseinheit (12) ist konfiguriert, die Ausgabeeinheit (13) zu veranlassen, die Menge des Rückstands gegenüber dem Nutzer auszugeben.

Das erfindungsgemäße System (S) kann einen oder mehrere Datenspeicher umfassen. Auf einem solchen Datenspeicher können Informationen zu Kulturpflanzen (z.B. bevorzugte Anbaubedingungen), zu Pflanzenschutzmitteln (z.B. bevorzugte Applikationsparameter), zum Klima eines Landes oder einer Region, zum Wetter eines Landes oder einer Region, zur Ausbreitung von Schadorganismen in einem Land oder einer Region und dergleichen gespeichert sein. Ein solcher Datenspeicher kann ein Bestandteil des ersten Computersystems (10), des zweiten Computersystems (20) und/oder eine separate Einheit sein, die mit dem ersten Computersystem (10) und/oder dem zweiten Computersystem (20) über ein Netzwerk verbunden sein kann.

Fig. 4 zeigt beispielhaft und schematisch in Form eines Ablaufdiagramms eine Ausführungsform des erfindungsgemäßen Verfahrens. Das Verfahren (100) umfasst die Schritte:
(110) Spezifizieren einer Kulturpflanze
(120) Spezifizieren von Anbauparametern zum Anbau der Kulturpflanze
(130) Spezifizieren eines Pflanzenschutzmittels
(140) Spezifizieren von Applikationsparametern zur Applikation des Pflanzenschutzmittels
(150) Berechnen einer Menge eines Rückstands des Pflanzenschutzmittels in und/oder auf den zum menschlichen und/oder tierischen Verzehr gedachten Teilen der Kulturpflanze vorzugsweise zum Zeitpunkt der Ernte
(160) Anzeigen der Menge des Rückstands gegenüber einem Nutzer.

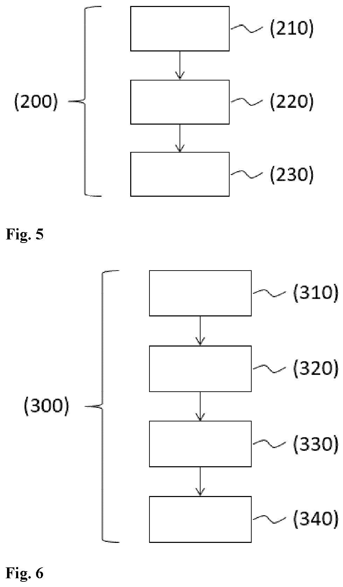

Fig. 5 zeigt beispielhaft und schematisch in Form eines Ablaufdiagramms die Schritte, die von einem Computersystem ausgeführt werden, auf dem das erfindungsgemäße Computerprogrammprodukt installiert ist. Die Schritte (200) umfassen:
(210) Empfangen folgender Informationen:
   ∘ angebaute Kulturpflanze
   ∘ Anbauparameter zum Anbau der Kulturpflanze
   ∘ zu verwendendes / verwendetes Pflanzenschutzmittel
   ∘ Applikationsparameter zur Applikation des Pflanzenschutzmittels
(220) Berechnen einer Menge eines Rückstands des Pflanzenschutzmittels in und/oder auf den zum menschlichen und/oder tierischen Verzehr gedachten Teilen der Kulturpflanze vorzugsweise zum Zeitpunkt der Ernte
(230) Anzeigen der Menge des Rückstands gegenüber einem Nutzer.

Fig. 6 zeigt beispielhaft und schematisch in Form eines Ablaufdiagramms die Schritte, die von einem Computersystem ausgeführt werden, auf dem eine bevorzugte Ausführungsform des Computerprogrammprodukts installiert ist. Die Schritte (300) umfassen:
(310) Empfangen folgender Eingabeinformationen von einem Nutzer:
   ∘ angebaute Kulturpflanze
   ∘ Anbauparameter zum Anbau der Kulturpflanze
   ∘ zu verwendendes / verwendetes Pflanzenschutzmittel
   ∘ Applikationsparameter zur Applikation des Pflanzenschutzmittels
(320) Übermitteln der Eingabeinformationen an ein zweites Computersystem
(330) Empfangen einer Menge eines Rückstands des Pflanzenschutzmittels in und/oder auf den zum menschlichen und/oder tierischen Verzehr gedachten Teilen der Kulturpflanze vorzugsweise zum Zeitpunkt der Ernte von dem zweiten Computersystem
(340) Anzeigen der Menge des Rückstands gegenüber einem Nutzer.

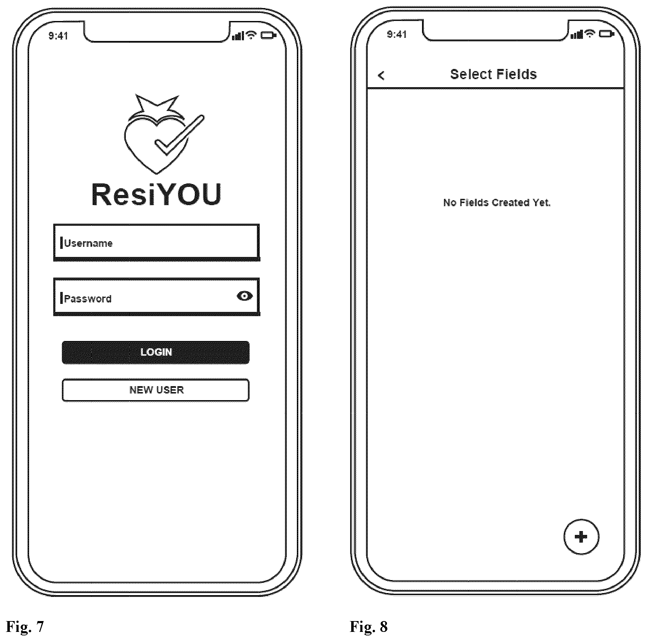

Fig. 7 bis Fig. 20 zeigen beispielhaft Anzeigen des erfindungsgemäßen Computerprogrammprodukts auf einem Bildschirm der erfindungsgemäßen Vorrichtung oder des erfindungsgemäßen Systems.

Fig. 7 zeigt beispielhaft eine Eingabemaske über die sich ein Nutzer mit seinem Namen (Username) und einem Passwort (Password) einloggen kann.

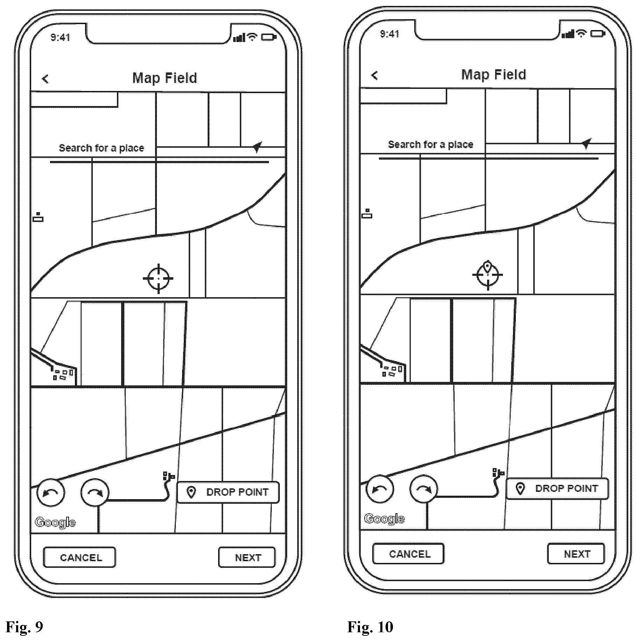

Nachdem sich der Nutzer eingeloggt hat, kann er aufgefordert werden, ein Feld, auf dem eine Kulturpflanze angebaut wird oder angebaut werden soll, zu spezifizieren. Fig. 8 zeigt eine Anzeige des erfindungsgemäßen Computerprogrammprodukts, die darauf hinweist, dass noch kein Feld spezifiziert ist ("No Fields Created Yet"). Durch Klicken der virtuellen Schaltfläche mit dem ⊕-Zeichen kann ein Nutzer einen Prozess zum Spezifizieren eines neuen Feldes starten. Der Prozess ist bildhaft in Fig. 9 bis Fig. 13 dargestellt.

Fig. 9 zeigt eine erste Anzeige zum Prozess zum Spezifizieren eines neuen Feldes. Es kann der Name eines Ortes (z.B. Land, Stand und/oder Straße) in ein Eingabefeld ("Search for a place") eingegeben werden. Im Hintergrund ist ein Ausschnitt der Erdoberfläche in einer Luftaufnahme oder in Form einer Landkarte dargestellt. Bei Eingeben des Namens eines Ortes wird ein Ausschnitt der Erdoberfläche gezeigt, der den Ort umfasst. Durch die von der Benutzung von Smartphones bekannten Fingerbewegungen kann der Nutzer den Ausschnitt verschieben, den Ausschnitt vergrößern (herauszoomen) oder den Ausschnitt verkleinern (hineinzoomen).

Ferner kann über eine virtuelle Schaltfläche ("Drop Point") ein Punkt spezifiziert werden, der in dem Feld oder an dem Rand des Feldes liegt. Bei Betätigen der virtuellen Schaltfläche wird ein solcher Punkt in die Mitte des Fadenkreuzes gesetzt. Dies ist in Fig. 10 gezeigt. Fig. 10 zeigt dieselbe Anzeige wie Fig. 9 mit dem Unterschied, dass nun ein erster Punkt des Feldes spezifiziert ist.

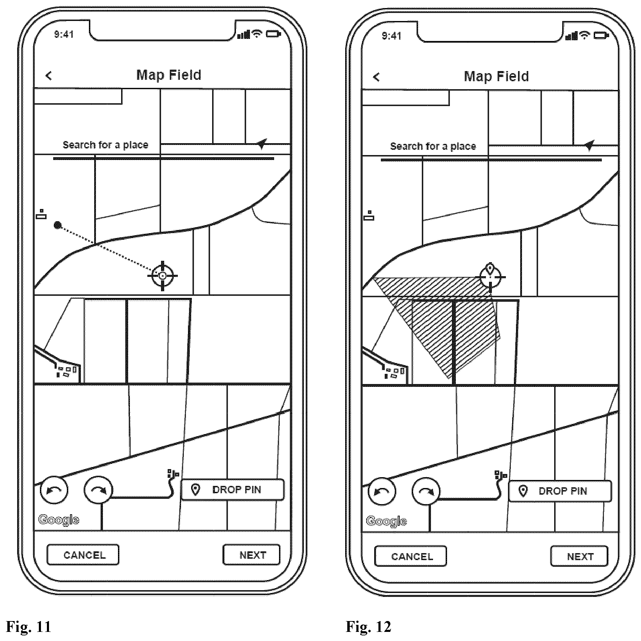

Ausgehend von dem spezifizierten Punkt kann ein Nutzer nun weitere Punkte des Feldes setzen (festlegen). Die Punkte werden durch Geraden miteinander verbunden. Dies ist in Fig. 11 und Fig. 12 dargestellt.

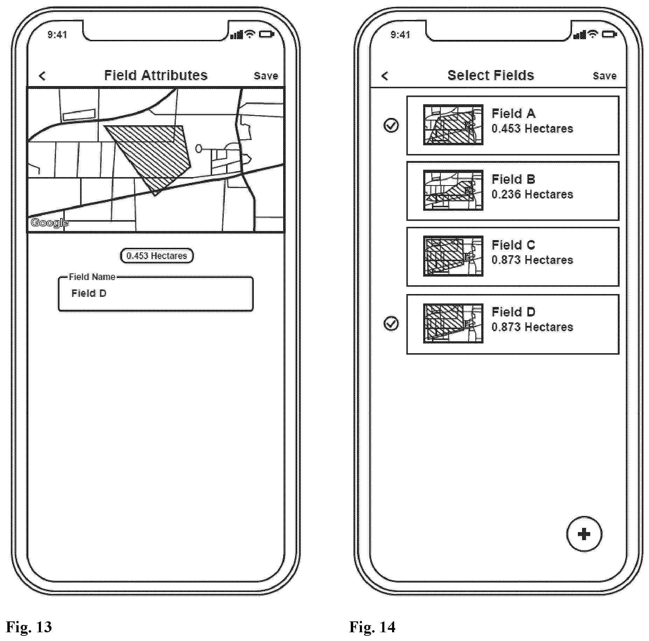

Das Computerprogramm ist so konfiguriert, dass es für ein spezifiziertes Feld automatisch die Größe errechnet. Fig. 13 zeigt das spezifizierte Feld und die errechnete Größe des Feldes ("0.453 Hectares"). Der Nutzer kann einen Namen für das Feld vergeben ("Field Name"), im vorliegenden Fall ist es der Name "Field D".

Fig. 14 zeigt eine weitere Anzeige des erfindungsgemäßen Computerprogramms. Es ist dargestellt, dass vier Felder mit den Namen "Field A", "Field B", "Field C", "Field D" angelegt worden sind. Der Nutzer kann diejenigen Felder auswählen, für die er eine Vorhersage einer Rückstandsmenge eines Pflanzenschutzmittels erzeugen lassen möchte. Die virtuelle Schaltfläche mit dem ⊕-Zeichen zeigt an, dass weitere Felder spezifiziert (im Computerprogramm angelegt) werden können.

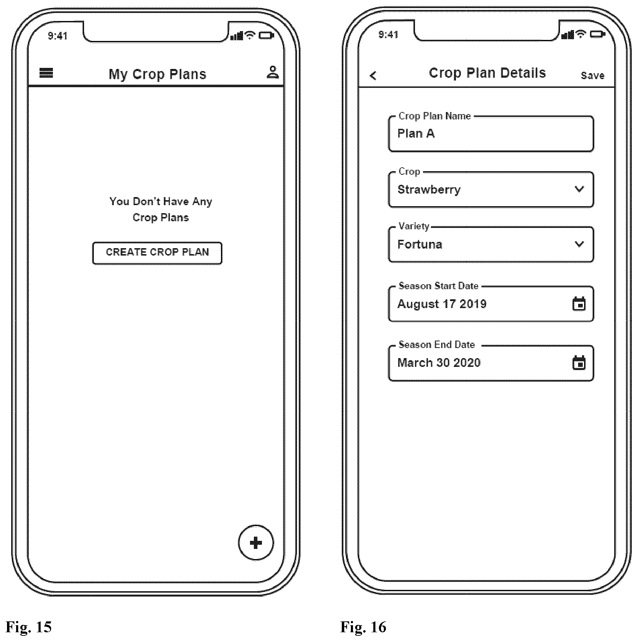

Fig. 15 zeigt eine weitere Anzeige des erfindungsgemäßen Computerprogramms. Das Computerprogramm zeigt dem Nutzer an, dass bisher keine Informationen zum Anbau einer Kulturpflanze und zur Applikation eines Pflanzenschutzmittels spezifiziert worden sind ("You Don't Have Any Crop Plans"). Durch Betätigen einer virtuellen Schaltfläche ("Create Crop Plan") kann der Nutzer einen Prozess zum Spezifizieren der entsprechenden Informationen starten. Der Prozess ist bildhaft in Fig. 16 dargestellt.

Fig. 16 zeigt eine Anzeige zum Erstellen eines Anbauplans für eine Kulturpflanze ("Crop Plan Details"). Die Anzeige umfasst eine Reihe von Eingabefeldern. In einem Eingabefeld kann der Nutzer dem jeweiligen Anbauplan einen Namen geben ("Crop Plan Name"). Im vorliegenden Fall hat der Nutzer als Namen "Plan A" eingegeben. In einem weiteren Eingabefeld kann der Nutzer die Kulturpflanze spezifizieren ("Crop"). Dies geschieht im vorliegenden Fall durch Auswahl eines Eintrags in einer Liste. Im vorliegenden Fall hat der Nutzer als Kulturpflanze Erdbeere ("Strawberry") ausgewählt. In einem weiteren Eingabefeld kann der Nutzer die Kulturpflanzensorte spezifizieren ("Variety"). Im vorliegenden Fall hat der Nutzer als Kulturpflanzensorte "Fortuna" ausgewählt. In einem weiteren Eingabefeld kann der Nutzer den Beginn der Anbauperiode spezifizieren ("Season Start Date"). Dies kann durch Eingabe eines Datums und/oder durch Auswahl eines Tages in einem virtuellen Kalender erfolgen. Im vorliegenden Fall hat der Nutzer als Beginn der Anbauperiode den 17. August 2019 spezifiziert. In einem weiteren Eingabefeld kann der Nutzer das Ende der Anbauperiode spezifizieren ("Season End Date"). Dies kann durch Eingabe eines Datums und/oder durch Auswahl eines Tages in einem virtuellen Kalender erfolgen. Im vorliegenden Fall hat der Nutzer als Ende der Anbauperiode den 30. März 2020 spezifiziert.

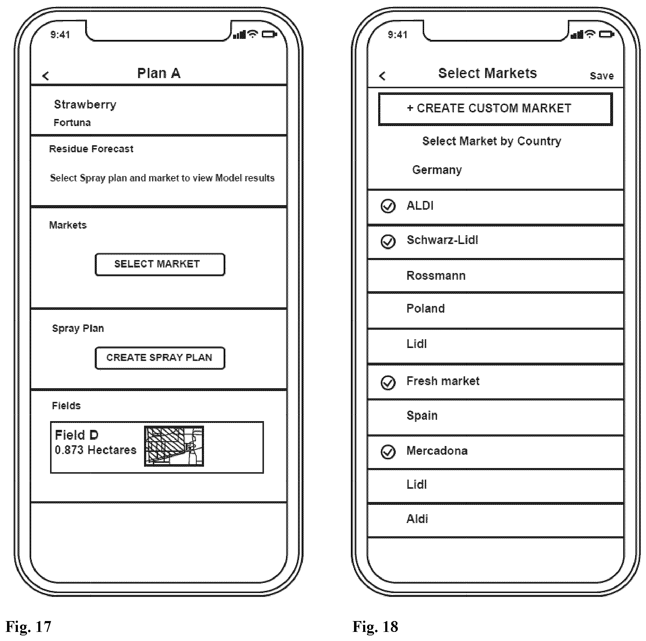

Fig. 17 zeigt eine weitere Anzeige des erfindungsgemäßen Computerprogramms. Fig. 17 zeigt einen Anbauplan mit dem Namen Plan A in einer Übersicht an. Der Anbauplan betrifft die Kulturpflanze Erdbeere ("Strawberry") der Sorte Fortuna. Es ist ein Feld mit dem Namen "Field D" spezifiziert, auf dem die Kulturpflanze angebaut wird / werden soll. Bevor das Computerprogramm eine Menge eines Rückstands eines Pflanzenschutzmittels berechnen (vorhersagen) kann, müssen noch ein oder mehrere Märkte ("Markets") und ein Applikationsprogramm für mindestens ein Pflanzenschutzmittel ("Spray Plan") spezifiziert werden. Durch Betätigen der jeweiligen virtuellen Schaltfläche kann der Nutzer einen Prozess zum Spezifizieren eines Marktes ("Select Market") oder einen Prozess zum Spezifizieren eines Applikationsprogramms für mindestens ein Pflanzenschutzmittel ("Create Spray Plan") starten.

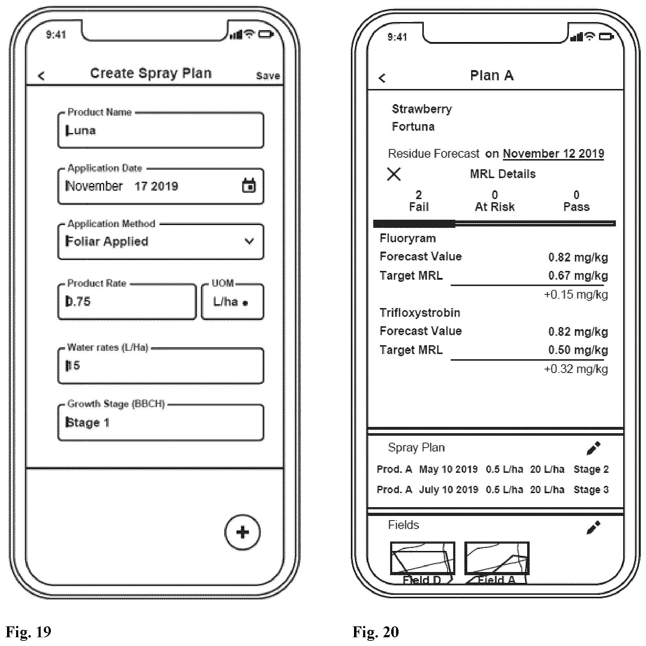

Der Prozess zum Spezifizieren eines Marktes ist bildhaft in Fig. 18 dargestellt. Der Prozess zum Spezifizieren eines Applikationsprogramms für mindestens ein Pflanzenschutzmittel ist bildhaft in Fig. 19 dargestellt.

Der Begriff "Markt" steht für eine Kombination aus einem Händler (oder eine Handelskette) und einem Land. Fig. 18 zeigt eine Übersicht von Märkten geordnet nach Ländern ("Market by Country"). Im vorliegenden Fall sind Handelsketten für drei Länder aufgelistet: für Deutschland ("Germany") die Handelsketten ALDI, Schwarz-Lidl und Rossmann, für Polen ("Poland") die Handelsketten Lidl und Fresh market, für Spanien ("Spain") die Handelsketten Mercadona, Lidl und ALDI. Der Nutzer kann eine oder mehrere Handelsketten durch Anklicken auswählen. Im vorliegenden Fall hat der Nutzer die folgenden Kombinationen ausgewählt: Deutschland: ALDI, Deutschland: Schwarz-Lidl, Polen: Fresh market, Spanien: Mercadona. Durch Betätigen der virtuellen Schaltfläche kann der Nutzer weitere Märkte anlegen ("Create Custom Market").

Fig. 19 zeigt eine Anzeige mit einer Reihe von Eingabefeldern zum Spezifizieren eines Applikationsprogramms für mindestens ein Pflanzenschutzmittel. Über ein erstes Eingabefeld kann das Pflanzenschutzmittel durch Eingeben eines Produktnamens ("Product Name") spezifiziert werden. Im vorliegenden Fall wurde der Produktname "Luna" eingegeben. In einem weiteren Eingabefeld kann der Nutzer das Datum der Applikation mit dem Pflanzenschutzmittel eingeben und/oder auswählen. Im vorliegenden Fall hat der Nutzer als Datum den 17. November 2019 spezifiziert. In einem weiteren Eingabefeld kann der Nutzer die Applikationsmethode ("Application Method") eingeben. Im vorliegenden Fall hat der Nutzer als Applikationsmethode Blattapplikation (Foliar Applied") eingegeben. In zwei weiteren Eingabefeldern kann der Nutzer die Menge an ausgebrachtem oder auszubringendem Pflanzenschutzmittel eingeben ("Product Rate", "UOM" (= Unit of Measure)). Im vorliegenden Fall hat der Nutzer eine Menge von 0,75 L/ha eingegeben. In einem weiteren Eingabefeld kann der Nutzer eine Menge an ausgebrachtem Wasser eingeben ("Water rates (L/ha)"). Im vorliegenden Fall hat der Nutzer eine Menge von 15 L/ha eingegeben. In einem weiteren Eingabefeld kann der Nutzer den Entwicklungsstand der Kulturpflanze (beim Ausbringen des Pflanzenschutzmittels) in Form des BBCH-Codes eingeben ("Growth Stage (BBCH)"). Im vorliegenden Fall hat der Nutzer den BBCH-Code 1 eingegeben ("Stage 1").

Fig. 20 zeigt eine Anzeige mit Ergebnissen einer Vorhersage. Die Ergebnisse werden für die Kulturpflanze Erdbeere ("Strawberry") der Sorte "Fortuna" vorhergesagt. Den Ergebnissen liegt ein Applikationsprogramm ("Plan A", "Spray Plan") zugrunde, bei dem zu zwei Zeitpunkten (10. Mai 2019 und 10. Juli 2019) jeweils 0,5 L/ha eines Pflanzenschutzmittels der Bezeichnung ("Prod. A") zusammen mit 20 L/ha Wasser appliziert worden sind. Zum 10. Mai 2019 lag/liegt die Kulturpflanze in dem Entwicklungsstadium mit dem BBCH-Code 2 ("Stage 2") vor; zum 10. Juli 2019 lag/liegt die Kulturpflanze in dem Entwicklungsstadium mit dem BBCH-Code 3 ("Stage 3") vor.

Das Pflanzenschutzmittel mit der Bezeichnung "Prod. A" umfasst zwei Wirkstoffe (Fluopyram, Trifloxystrobin). Als Zeitpunkt, für den die Vorhersage erfolgt, ist der 12. November 2019 angegeben ("Residue Forecast on November 12 2019"). Die berechnete (vorhergesagte) Rückstandsmenge an Fluopyram beträgt 0,82 mg/kg. Die berechnete (vorhergesagte) Rückstandsmenge an Trifloxystrobin beträgt 0,82 mg/kg. Beide Rückstandsmengen liegen oberhalb des MRL-Werts (0,67 mg/kg für Fluopyram, 0,5 mg/kg für Trifloxystrobin).

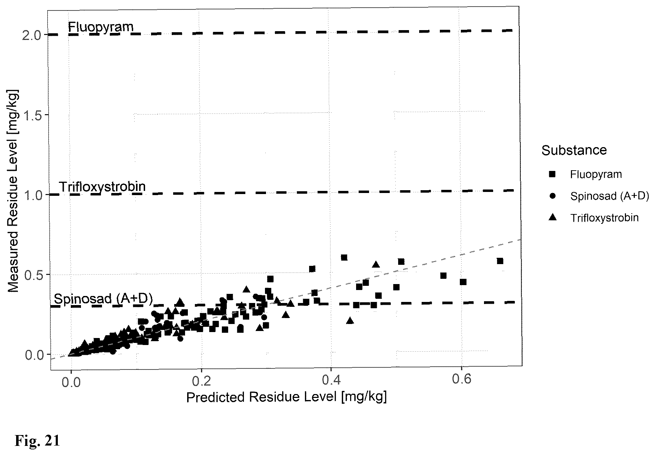

Fig. 21 zeigt in Form einer Grafik einen Vergleich zwischen gemessenen Rückstandsmengen (y-Achse (Ordinate) "*Measured Residues Level [mg*/*kg]*") und erfindungsgemäß berechneten (vorhergesagten) Rückstandsmengen (X-Achse (Abszisse) "*Predicted Residue Level [mg*/*kg]*) für drei Pflanzenschutzmittel (Fluopyram, Spinosad (A+D) und Trifloxystrobin). Jeder Messpunkt in der Grafik steht für eine gemessene und eine erfindungsgemäß berechnete Rückstandsmenge. Die Messungen und Berechnungen wurden für Erdbeeren, die unter verschiedenen Bedingungen angebaut wurden, ermittelt. Die Vorhersage erfolgte mit Hilfe eines nicht-linearen Regressionsmodells mit den folgenden Eingangsgrößen:
- verwendetes Pflanzenschutzmittel
- Zahl der Applikationen des Pflanzenschutzmittels und applizierte Mengen
- Zeitspanne zwischen der letzten Applikation und dem Erntezeitpunkt
- durchschnittliche Lufttemperatur an den jeweiligen Tagen der Applikationen
- durchschnittliche Luftfeuchte an den jeweiligen Tagen der Applikationen
- akkumulierte Strahlungsenergie (Sonnenstrahlung oder künstliche Beleuchtung) an den jeweiligen Tagen der Applikationen
- durchschnittlicher Durchmesser und durchschnittliche Höhe der Kulturpflanzen (Erdbeeren) an den jeweiligen Applikationstagen.

Man erkennt in Fig. 21 eine eindeutige Korrelation zwischen vorhergesagten und gemessenen Rückstandsmengen. Als horizontale Linien sind in der Grafik Höchstmengen für die Pflanzenschutzmittel angegeben. Die Höchstmengen für Fluopyran und Trifloxystrobin werden in der gezeigten Grafik nicht überschritten; die Höchstmenge für Spinosad (A+D) ist in einigen Fällen überschritten.

## Patentansprüche

1. System (S) umfassend
- ein erstes Computersystem (10) umfassend eine Eingabeeinheit (11), eine erste Steuer- und Berechnungseinheit (12), eine erste Sende- und Empfangseinheit (14) und eine Ausgabeeinheit (13),
- ein zweites Computersystem (20) umfassend eine zweite Steuer- und Berechnungseinheit (22) und eine zweite Sende- und Empfangseinheit (24),
wobei die erste Steuer- und Berechnungseinheit (12) konfiguriert ist, die Eingabeeinheit (11) zu veranlassen, folgende Eingabeinformationen zu empfangen und/oder zu ermitteln:
- Informationen zu einer Kulturpflanze, die angebaut wird,
- Informationen zum Anbau der Kulturpflanze,
- Informationen zu einem Pflanzenschutzmittel, das beim Anbau der Kulturpflanze verwendet worden ist und/oder dessen Verwendung geplant ist, wobei es sich bei dem Pflanzenschutzmittel um ein Herbizid, ein Fungizid oder ein anderes Pestizid handelt,
- Informationen zur Applikation des Pflanzenschutzmittels,
wobei die erste Steuer- und Berechnungseinheit (12) konfiguriert ist, die erste Sende- und Empfangseinheit (14) zu veranlassen, die Eingabeinformationen über ein Netzwerk an das zweite Computersystem (20) zu übermitteln,
wobei die zweite Steuer- und Berechnungseinheit (22) konfiguriert ist, die zweite Sende- und Empfangseinheit (24) zu veranlassen, die Eingabeinformationen über das Netzwerk zu empfangen, wobei die zweite Steuer- und Berechnungseinheit (22) konfiguriert ist, eine Menge eines Rückstands des Pflanzenschutzmittels in und/oder auf den zum menschlichen und/oder tierischen Verzehr gedachten Teilen der Kulturpflanze vorzugsweise zum Zeitpunkt der Ernte auf Basis der Eingabeinformationen zu berechnen,
wobei das Berechnen der Menge des Rückstands des Pflanzenschutzmittels mindestens anhand der folgenden Eingabeinformationen erfolgt:
- angebaute Kulturpflanze
- zu verwendendes oder verwendetes Pflanzenschutzmittel
- Aufwandmenge des zu verwendenden oder verwendeten Pflanzenschutzmittels
- Menge der vorliegenden Biomasse und/oder Fruchtmasse der angebauten Kulturpflanze zum Zeitpunkt der Applikation des Pflanzenschutzmittels
- Länge der Zeitspanne zwischen dem Zeitpunkt der Applikation und dem Zeitpunkt der Ernte,
wobei das Berechnen der Menge des Rückstands des Pflanzenschutzmittels mit einem Regressionsmodell erfolgt, das mit empirischen Daten trainiert worden ist, die Menge des Rückstands des Pflanzenschutzmittels anhand von Informationen vorherzusagen, wobei die Informationen umfassen:
- angebaute Kulturpflanze
- Land oder Region, in der die Kulturpflanze angebaut wird oder Menge der vorliegenden Biomasse und/oder Fruchtmasse der angebauten Kulturpflanze zum Zeitpunkt der Applikation des Pflanzenschutzmittels
- zu verwendendes oder verwendetes Pflanzenschutzmittel
- Aufwandmenge des zu verwendenden oder verwendeten Pflanzenschutzmittels
- Länge der Zeitspanne zwischen dem Zeitpunkt der Applikation und dem Zeitpunkt der Ernte,
wobei die zweite Steuer- und Berechnungseinheit (22) konfiguriert ist, die zweite Sende- und Empfangseinheit (24) zu veranlassen, die Menge des Rückstands über das Netzwerk an das erste Computersystem (10) zu übermitteln,
wobei die erste Steuer- und Berechnungseinheit (12) konfiguriert ist, die erste Sende- und Empfangseinheit (14) zu veranlassen, die Menge des Rückstands über das Netzwerk zu empfangen, wobei die erste Steuer- und Berechnungseinheit (12) konfiguriert ist, die Ausgabeeinheit (13) zu veranlassen die Menge des Rückstands gegenüber einem Nutzer auszugeben.

2. System (S) gemäß Anspruch 1, wobei das Regressionsmodell mittels überwachten Lernens erstellt wird.

3. System (S) gemäß Anspruch 1, wobei das Regressionsmodell ein nicht-lineares Regressionsmodell ist.

4. System (S) gemäß einem der Ansprüche 1 bis 3, wobei die Menge des Rückstands gegenüber dem Nutzer als Anteil eines von einer staatlichen Behörde und/oder einem Händler vorgegebenen Maximalmenge angezeigt wird.

5. System (S) gemäß einem der Ansprüche 1 bis 4, wobei die Kulturpflanze aus der folgenden Liste ausgewählt ist: Erdbeere, Tomate, Gurke, Paprika, Radies, Rettich, Kohlrabi, Möhre, Sellerie, Fenchel, Patinake, Erbse, Bohne, Spargel, Spinat, Mangold, Artischocke, Schwarzwurzel, Kopfsalat, Eissalat, Schnittsalat, Endivie, Cicoree, Aubergine, Kürbis, Melone, Zucchini, Feldsalat, Zuckerrübe, Rhabarber, Weißkohl, Rotkohl, Grünkohl, Rosenkohl, Blumenkohl, Broccoli, Himbeere, Brombeere, Holunder, Kirsche, Apfel, Birne, Weintraube, Pflaume.

6. System (S) gemäß einem der Ansprüche 1 bis 5, wobei das Pflanzenschutzmittel ein Fungizid ist.

## Claims

1. A system (S) comprising
- a first computer system (10) comprising an input unit (11), a first control and computation unit (12), a first transmitting and receiving unit (14) and an output unit (13),
- a second computer system (20) comprising a second control and computation unit (22) and a second transmitting and receiving unit (24),
wherein the first control and computation unit (12) is configured to cause the input unit (11) to receive and/or determine the following input information:
- information on a crop plant which is cultivated,
- information on the cultivation of the crop plant,
- information on a plant protection agent which has been used and/or whose use is planned in the cultivation of the crop plant, wherein the plant protection agent is a herbicide, a fungicide or another pesticide,
- information on the application of the plant protection agent,
wherein the first control and computation unit (12) is configured to cause the first transmitting and receiving unit (14) to transmit the input information via a network to the second computer system (20),
wherein the second control and computation unit (22) is configured to cause the second transmitting and receiving unit (24) to receive the input information via the network,
wherein the second control and computation unit (22) is configured to calculate, on the basis of the input information, an amount of a residue of the plant protection agent in and/or on the parts of the crop plant intended for human and/or animal consumption, preferably at the time of harvest,
wherein the calculation of the amount of the residue of the plant protection agent is carried out at least on the basis of the following input information:
- cultivated crop plant
- plant protection agent to be used or used
- application rate of the plant protection agent to be used or used
- amount of biomass and/or fruit mass of the cultivated crop plant present at the time of application of the plant protection agent
- length of the time period between the time of application and the time of harvest,
wherein the calculation of the amount of the residue of the plant protection agent is carried out with a regression model which has been trained with empirical data to predict the amount of the residue of the plant protection agent on the basis of information, wherein the information comprises:
- cultivated crop plant
- country or region in which the crop plant is cultivated or amount of biomass and/or fruit mass of the cultivated crop plant present at the time of application of the plant protection agent
- plant protection agent to be used or used
- application rate of the plant protection agent to be used or used
- length of the time period between the time of application and the time of harvest,
wherein the second control and computation unit (22) is configured to cause the second transmitting and receiving unit (24) to transmit the amount of the residue via the network to the first computer system (10),
wherein the first control and computation unit (12) is configured to cause the first transmitting and receiving unit (14) to receive the amount of the residue via the network,
wherein the first control and computation unit (12) is configured to cause the output unit (13) to output the amount of the residue to a user.

2. The system (S) according to claim 1, wherein the regression model is created by means of supervised learning.

3. The system (S) according to claim 1, wherein the regression model is a non-linear regression model.

4. The system (S) according to any one of claims 1 to 3, wherein the amount of the residue is displayed to the user as a proportion of a maximum amount specified by a governmental authority and/or a trader.

5. The system (S) according to any one of claims 1 to 4, wherein the crop plant is selected from the following list: strawberry, tomato, cucumber, sweet pepper, radish, white radish, kohlrabi, carrot, celery, fennel, parsnip, pea, bean, asparagus, spinach, chard, artichoke, black salsify, butterhead lettuce, iceberg lettuce, leaf lettuce, endive, chicory, aubergine, pumpkin, melon, courgette, lamb's lettuce, sugar beet, rhubarb, white cabbage, red cabbage, kale, Brussels sprouts, cauliflower, broccoli, raspberry, blackberry, elder, cherry, apple, pear, grape, plum.

6. The system (S) according to any one of claims 1 to 5, wherein the plant protection agent is a fungicide.

## Revendications

1. Système (S) comprenant
- un premier système informatique (10) comprenant une unité d'entrée (11), une première unité de commande et de calcul (12), une première unité d'émission et de réception (14) et une unité de sortie (13),
- un second système informatique (20) comprenant une seconde unité de commande et de calcul (22) et une seconde unité d'émission et de réception (24),
dans lequel la première unité de commande et de calcul (12) est configurée pour amener l'unité d'entrée (11) à recevoir et/ou à déterminer les informations d'entrée suivantes :
- des informations relatives à une plante cultivée qui est mise en culture,
- des informations relatives à la culture de la plante cultivée,
- des informations relatives à un agent phytosanitaire qui a été utilisé et/ou dont l'utilisation est prévue lors de la culture de la plante cultivée, l'agent phytosanitaire étant un herbicide, un fongicide ou un autre pesticide,
- des informations relatives à l'application de l'agent phytosanitaire,
dans lequel la première unité de commande et de calcul (12) est configurée pour amener la première unité d'émission et de réception (14) à transmettre les informations d'entrée par l'intermédiaire d'un réseau au second système informatique (20),
dans lequel la seconde unité de commande et de calcul (22) est configurée pour amener la seconde unité d'émission et de réception (24) à recevoir les informations d'entrée par l'intermédiaire du réseau,
dans lequel la seconde unité de commande et de calcul (22) est configurée pour calculer, sur la base des informations d'entrée, une quantité d'un résidu de l'agent phytosanitaire dans et/ou sur les parties de la plante cultivée destinées à la consommation humaine et/ou animale, de préférence au moment de la récolte,
dans lequel le calcul de la quantité du résidu de l'agent phytosanitaire est effectué au moins sur la base des informations d'entrée suivantes :
- plante cultivée mise en culture
- agent phytosanitaire à utiliser ou utilisé
- dose d'application de l'agent phytosanitaire à utiliser ou utilisé
- quantité de biomasse et/ou de masse de fruits de la plante cultivée mise en culture présente au moment de l'application de l'agent phytosanitaire
- durée de l'intervalle de temps entre le moment de l'application et le moment de la récolte,
dans lequel le calcul de la quantité du résidu de l'agent phytosanitaire est effectué au moyen d'un modèle de régression qui a été entraîné avec des données empiriques à prédire la quantité du résidu de l'agent phytosanitaire sur la base d'informations, les informations comprenant :
- plante cultivée mise en culture
- pays ou région dans lequel ou dans laquelle la plante cultivée est mise en culture ou quantité de biomasse et/ou de masse de fruits de la plante cultivée mise en culture présente au moment de l'application de l'agent phytosanitaire
- agent phytosanitaire à utiliser ou utilisé
- dose d'application de l'agent phytosanitaire à utiliser ou utilisé
- durée de l'intervalle de temps entre le moment de l'application et le moment de la récolte,
dans lequel la seconde unité de commande et de calcul (22) est configurée pour amener la seconde unité d'émission et de réception (24) à transmettre la quantité du résidu par l'intermédiaire du réseau au premier système informatique (10),
dans lequel la première unité de commande et de calcul (12) est configurée pour amener la première unité d'émission et de réception (14) à recevoir la quantité du résidu par l'intermédiaire du réseau,
dans lequel la première unité de commande et de calcul (12) est configurée pour amener l'unité de sortie (13) à délivrer en sortie la quantité du résidu à un utilisateur.

2. Système (S) selon la revendication 1, dans lequel le modèle de régression est créé au moyen d'un apprentissage supervisé.

3. Système (S) selon la revendication 1, dans lequel le modèle de régression est un modèle de régression non linéaire.

4. Système (S) selon l'une quelconque des revendications 1 à 3, dans lequel la quantité du résidu est affichée à l'utilisateur en tant que fraction d'une quantité maximale prescrite par une autorité publique et/ou un négociant.

5. Système (S) selon l'une quelconque des revendications 1 à 4, dans lequel la plante cultivée est choisie dans la liste suivante : fraise, tomate, concombre, poivron, radis, radis long, chou-rave, carotte, céleri, fenouil, panais, pois, haricot, asperge, épinard, blette, artichaut, scorsonère, laitue pommée, laitue iceberg, laitue à couper, chicorée scarole, endive, aubergine, courge, melon, courgette, mâche, betterave sucrière, rhubarbe, chou blanc, chou rouge, chou frisé, chou de Bruxelles, chou-fleur, brocoli, framboise, mûre, sureau, cerise, pomme, poire, raisin, prune.

6. Système (S) selon l'une quelconque des revendications 1 à 5, dans lequel l'agent phytosanitaire est un fongicide.
